# EUROPEAN PATENT SPECIFICATION

(11) **EP 3 430 162 B1**
(45) Date of publication and mention of the grant of the patent: **29.06.2022**
(21) Application number: 17712443.5
(22) Date of filing: 17.03.2017
(51) Int. Cl.: C12Q 1/68

(54) **METHYLATION MARKERS FOR PANCREATIC CANCER**
METHYLIERUNGSMARKER FÜR BAUCHSPEICHELDRÜSENKREBS
MARQUEUR DE METHYLATION POUR LE CANCER DU PANCRÉAS

(30) Priority: 18.03.2016 EP 16161073
(43) Date of publication of application: 23.01.2019
(73) Proprietor: Region Nordjylland, Aalborg University Hospital, 9220 Aalborg Ø (DK)
(72) Inventor: THORLACIUS-USSING, Ole, 9000 Aalborg (DK); HENRIKSEN, Stine Dam, 9260 Gistrup (DK); KRARUP, Henrik, 9440 Pandrup (DK); MADSEN, Poul, 9400 Nørresundby (DK)
(74) Representative: Høiberg P/S
(86) International application number: PCT/EP2017/056393
(87) International publication number: WO 2017/158158

(56) References cited:
- WO-A2-2004/083399
- WO-A2-2008/063655
- US-A1- 2008 261 217
- HENRIKSEN S D ET AL: "DNA hypermethylation as a blood-based marker for pancreatic cancer: A literature review", PANCREAS 20151001 LIPPINCOTT WILLIAMS AND WILKINS USA, vol. 44, no. 7, 1 October 2015 (2015-10-01), pages 1036-1045, XP008181224, ISSN: 0885-3177
- DATABASE BIOSIS [Online] BIOSCIENCES INFORMATION SERVICE, PHILADELPHIA, PA, US; April 2010 (2010-04), MANGOLD KATHY A ET AL: "Methylated cell-free DNA in plasma as an early biomarker of pancreatic cancer", XP008181223, Database accession no. PREV201000525773 & PROCEEDINGS OF THE AMERICAN ASSOCIATION FOR CANCER RESEARCH ANNUAL MEETING, vol. 51, April 2010 (2010-04), page 1193, 101ST ANNUAL MEETING OF THE AMERICAN-ASSOCIATION-FOR-CANCER-RESEARCH; WASHINGTON, DC, USA; APRIL 17 -21, 2010 ISSN: 0197-016X
- NIKOLIC ALEKSANDRA: "Noninvasive Early Markers in Pancreatic Cancer", CANCER BIOMARKERS: MINIMAL AND NONINVASIVE EARLY DIAGNOSIS AND PROGNOSIS CRC PRESS-TAYLOR & FRANCIS GROUP, 6000 BROKEN SOUND PARKWAY NW, STE 300, BOCA RATON, FL 33487-2742 USA, 2014, pages 383-412, XP008181222,
- DANIEL NEUREITER ET AL: "Epigenetics and pancreatic cancer: Pathophysiology and novel treatment aspects", WORLD JOURNAL OF GASTROENTEROLOGY, vol. 20, no. 24, 1 January 2014 (2014-01-01), page 7830, XP055295961, CN ISSN: 1007-9327, DOI: 10.3748/wjg.v20.i24.7830
- TAN A C ET AL: "Characterizing DNA methylation patterns in pancreatic cancer genome", MOLECULAR ONCOLOGY, ELSEVIER, AMSTERDAM, NL, vol. 3, no. 5-6, 1 December 2009 (2009-12-01), pages 425-438, XP026770016, ISSN: 1574-7891, DOI: 10.1016/J.MOLONC.2009.03.004 [retrieved on 2009-04-22]

### SEQUENCE LISTING

<110> Idéklinikken - Region Nordjylland
<120> Pancreatic cancer
<130> P4150EP00
<160> 143
<170> PatentIn version 3.5
<210> 1
   <211> 21
   <212> DNA
   <213> Human
<400> 1
   gcgtcgagat gttgttttgt c 21
<210> 2
   <211> 24
   <212> DNA
   <213> Human
<400> 2
   ccaaaaccaa aacgataaac aacg 24
<210> 3
   <211> 27
   <212> DNA
   <213> Human
<400> 3
   cgatcgacca acgcgcacta acgatcg 27
<210> 4
   <211> 25
   <212> DNA
   <213> Human
<400> 4
   agagtatata agtcgtttta ggagc 25
<210> 5
   <211> 24
   <212> DNA
   <213> Human
<400> 5
   ccaaaaccaa aacgataaac aacg 24
<210> 6
   <211> 17
   <212> DNA
   <213> Human
<400> 6
   agtgcgggtc gggaagc 17
<210> 7
   <211> 19
   <212> DNA
   <213> Human
<400> 7
   aatcgacgaa ctcccgacg 19
<210> 8
   <211> 25
   <212> DNA
   <213> Human
<400> 8
   cgcgatcgtt ggatgcggaa tcgcg 25
<210> 9
   <211> 17
   <212> DNA
   <213> Human
<400> 9
   attgcggagt gcgggtc 17
<210> 10
   <211> 19
   <212> DNA
   <213> Human
<400> 10
   aatcgacgaa ctcccgacg 19
<210> 11
   <211> 23
   <212> DNA
   <213> Human
<400> 11
   tggttttttg gcgttaaaat gtc 23
<210> 12
   <211> 18
   <212> DNA
   <213> Human
<400> 12
   aaataacttc ccccgccg 18
<210> 13
   <211> 30
   <212> DNA
   <213> Human
<400> 13
   cgcgatctcg tccaaccgcc gaatatcgcg 30
<210> 14
   <211> 23
   <212> DNA
   <213> Human
<400> 14
   tggttttttg gcgttaaaat gtc 23
<210> 15
   <211> 27
   <212> DNA
   <213> Human
<400> 15
   catctcttta ataacattaa ctaaccg 27
<210> 16
   <211> 18
   <212> DNA
   <213> Human
<400> 16
   ggagttgatt ggttgcgc 18
<210> 17
   <211> 16
   <212> DNA
   <213> Human
<400> 17
   cgcgacacta actccg 16
<210> 18
   <211> 24
   <212> DNA
   <213> Human
<400> 18
   cgcgatggtt cggtcgtaat cgcg 24
<210> 19
   <211> 18
   <212> DNA
   <213> Human
<400> 19
   gaggcgattg gttttcgc 18
<210> 20
   <211> 16
   <212> DNA
   <213> Human
<400> 20
   cgcgacacta actccg 16
<210> 21
   <211> 24
   <212> DNA
   <213> Human
<400> 21
   gtttcggttt tagtttcgta tttc 24
<210> 22
   <211> 21
   <212> DNA
   <213> Human
<400> 22
   cgactcctac gtctaaacgc g 21
<210> 23
   <211> 22
   <212> DNA
   <213> Human
<400> 23
   cgcgatccgc acgtccatcg cg 22
<210> 24
   <211> 24
   <212> DNA
   <213> Human
<400> 24
   gtttcggttt tagtttcgta tttc 24
<210> 25
   <211> 20
   <212> DNA
   <213> Human
<400> 25
   ccctaaaaac gactcctacg 20
<210> 26
   <211> 16
   <212> DNA
   <213> Human
<400> 26
   gggaggcgtt gaagtc 16
<210> 27
   <211> 21
   <212> DNA
   <213> Human
<400> 27
   gtacttcgct aactttaaac g 21
<210> 28
   <211> 24
   <212> DNA
   <213> Human
<400> 28
   cgcgattcgt tcggttcgct cgcg 24
<210> 29
   <211> 16
   <212> DNA
   <213> Human
<400> 29
   gggaggcgtt gaagtc 16
<210> 30
   <211> 20
   <212> DNA
   <213> Human
<400> 30
   aataaactca aactcccccg 20
<210> 31
   <211> 21
   <212> DNA
   <213> Human
<400> 31
   tttcgagtat tcgtttatag c 21
<210> 32
   <211> 21
   <212> DNA
   <213> Human
<400> 32
   tttcttcctc cgatactaac g 21
<210> 33
   <211> 27
   <212> DNA
   <213> Human
<400> 33
   cgacgtgaaa gatatcgcgg tacttcg 27
<210> 34
   <211> 26
   <212> DNA
   <213> Human
<400> 34
   tgttcggagt taatagtatt tttttc 26
<210> 35
   <211> 21
   <212> DNA
   <213> Human
<400> 35
   tttcttcctc cgatactaac g 21
<210> 36
   <211> 21
   <212> DNA
   <213> Human
<400> 36
   gggtatcgtc ggggtagatt c 21
<210> 37
   <211> 21
   <212> DNA
   <213> Human
<400> 37
   tcgaccaatc caaccgaaac g 21
<210> 38
   <211> 25
   <212> DNA
   <213> Human
<400> 38
   cgcgacgaat acgttccgaa tcgcg 25
<210> 39
   <211> 21
   <212> DNA
   <213> Human
<400> 39
   agtagggttt gtttgggtat c 21
<210> 40
   <211> 21
   <212> DNA
   <213> Human
<400> 40
   tcgaccaatc caaccgaaac g 21
<210> 41
   <211> 21
   <212> DNA
   <213> Human
<400> 41
   gggattgtat ttgttttcgt c 21
<210> 42
   <211> 17
   <212> DNA
   <213> Human
<400> 42
   acgcaacgca tatcccg 17
<210> 43
   <211> 25
   <212> DNA
   <213> Human
<400> 43
   cgcgatgaac gacccgacga tcgcg 25
<210> 44
   <211> 23
   <212> DNA
   <213> Human
<400> 44
   gttttgggat tgtatttgtt ttc 23
<210> 45
   <211> 17
   <212> DNA
   <213> Human
<400> 45
   acgcaacgca tatcccg 17
<210> 46
   <211> 21
   <212> DNA
   <213> Human
<400> 46
   tcgtggtaac ggaaaagcgc g 21
<210> 47
   <211> 20
   <212> DNA
   <213> Human
<400> 47
   ccgtccaaaa aatctcaacg 20
<210> 48
   <211> 22
   <212> DNA
   <213> Human
<400> 48
   cgatcggcgg cgtgagcgta cg 22
<210> 49
   <211> 22
   <212> DNA
   <213> Human
<400> 49
   gttttttggt tttcgtggta ac 22
<210> 50
   <211> 21
   <212> DNA
   <213> Human
<400> 50
   aaacccacaa cctatccccc g 21
<210> 51
   <211> 20
   <212> DNA
   <213> Human
<400> 51
   cgacgtttta gtttcgagtc 20
<210> 52
   <211> 25
   <212> DNA
   <213> Human
<400> 52
   cgcttcctaa aaccaaaaat tctcg 25
<210> 53
   <211> 23
   <212> DNA
   <213> Human
<400> 53
   cgatcggtgg tcgggttcga tcg 23
<210> 54
   <211> 17
   <212> DNA
   <213> Human
<400> 54
   gcgatgggtt tgtgcgc 17
<210> 55
   <211> 21
   <212> DNA
   <213> Human
<400> 55
   gaaaaaccga ttacgcatac g 21
<210> 56
   <211> 20
   <212> DNA
   <213> Human
<400> 56
   gatatgttgg gatagttcgc 20
<210> 57
   <211> 22
   <212> DNA
   <213> Human
<400> 57
   gcactcttcc gaaaacgaaa cg 22
<210> 58
   <211> 29
   <212> DNA
   <213> Human
<400> 58
   cgcgatcgta tcgtttgcga tttatcgcg 29
<210> 59
   <211> 20
   <212> DNA
   <213> Human
<400> 59
   gatatgttgg gatagttcgc 20
<210> 60
   <211> 19
   <212> DNA
   <213> Human
<400> 60
   aaaaaactcc gcacttccg 19
<210> 61
   <211> 24
   <212> DNA
   <213> Human
<400> 61
   gtttagtatt tattttcgaa gttc 24
<210> 62
   <211> 15
   <212> DNA
   <213> Human
<400> 62
   cctccgcgcg acccg 15
<210> 63
   <211> 32
   <212> DNA
   <213> Human
<400> 63
   cgacgtattt agttgcgcgt tgatcgacgt cg 32
<210> 64
   <211> 24
   <212> DNA
   <213> Human
<400> 64
   gtttagtatt tattttcgaa gttc 24
<210> 65
   <211> 18
   <212> DNA
   <213> Human
<400> 65
   gccgaaaacg cttcctcg 18
<210> 66
   <211> 21
   <212> DNA
   <213> Human
<400> 66
   atatttatcg cgttttcgtt c 21
<210> 67
   <211> 24
   <212> DNA
   <213> Human
<400> 67
   acgaacctaa taaacataac tacg 24
<210> 68
   <211> 31
   <212> DNA
   <213> Human
<400> 68
   cgacgtgttc gcgttatcgt cgtcgacgtc g 31
<210> 69
   <211> 20
   <212> DNA
   <213> Human
<400> 69
   gaggttttcg cgttagagac 20
<210> 70
   <211> 24
   <212> DNA
   <213> Human
<400> 70
   acgaacctaa taaacataac tacg 24
<210> 71
   <211> 16
   <212> DNA
   <213> Human
<400> 71
   cggaggtagc ggtaac 16
<210> 72
   <211> 19
   <212> DNA
   <213> Human
<400> 72
   cgatacgaac gaacgaacg 19
<210> 73
   <211> 32
   <212> DNA
   <213> Human
<400> 73
   cgcgatttcg gtttcgtcgg attcgtatcg cg 32
<210> 74
   <211> 17
   <212> DNA
   <213> Human
<400> 74
   gggtcggttt tttcggc 17
<210> 75
   <211> 17
   <212> DNA
   <213> Human
<400> 75
   acgaatcccg acgaacg 17
<210> 76
   <211> 16
   <212> DNA
   <213> Human
<400> 76
   agtggagacg gcgttc 16
<210> 77
   <211> 20
   <212> DNA
   <213> Human
<400> 77
   cttactacgc taacccaacg 20
<210> 78
   <211> 30
   <212> DNA
   <213> Human
<400> 78
   cgtcgagcgg gtgaggttcg cgtatcgacg 30
<210> 79
   <211> 18
   <212> DNA
   <213> Human
<400> 79
   tagcgttgga gtggagac 18
<210> 80
   <211> 18
   <212> DNA
   <213> Human
<400> 80
   ccaaccccac ttactacg 18
<210> 81
   <211> 21
   <212> DNA
   <213> Human
<400> 81
   tttttcggag gcgataagtt c 21
<210> 82
   <211> 18
   <212> DNA
   <213> Human
<400> 82
   cgaacccgac tcttaacg 18
<210> 83
   <211> 31
   <212> DNA
   <213> Human
<400> 83
   cgcgattgtc ggtcgtcgtt aaagtatcgc g 31
<210> 84
   <211> 20
   <212> DNA
   <213> Human
<400> 84
   cgttttcgtt cgtcgtttgc 20
<210> 85
   <211> 18
   <212> DNA
   <213> Human
<400> 85
   cgaacccgac tcttaacg 18
<210> 86
   <211> 19
   <212> DNA
   <213> Human
<400> 86
   gtttttcgga gttgcgcgc 19
<210> 87
   <211> 21
   <212> DNA
   <213> Human
<400> 87
   ccgaaaaact aacaaccgac g 21
<210> 88
   <211> 36
   <212> DNA
   <213> Human
<400> 88
   cgacgtttgt agcgtttcgt tcgcgttgtt acgtcg 36
<210> 89
   <211> 19
   <212> DNA
   <213> Human
<400> 89
   gtttttcgga gttgcgcgc 19
<210> 90
   <211> 21
   <212> DNA
   <213> Human
<400> 90
   ctcttcgcta aatacgactc g 21
<210> 91
   <211> 18
   <212> DNA
   <213> Human
<400> 91
   gttgatttga tcgtcggc 18
<210> 92
   <211> 19
   <212> DNA
   <213> Human
<400> 92
   ctcgcctaca aaaaccacg 19
<210> 93
   <211> 30
   <212> DNA
   <213> Human
<400> 93
   cgcgatgccc gaccgatctc ctaaatcgcg 30
<210> 94
   <211> 22
   <212> DNA
   <213> Human
<400> 94
   gtttatacga gttgatttga tc 22
<210> 95
   <211> 18
   <212> DNA
   <213> Human
<400> 95
   cttaacctaa cctcctcg 18
<210> 96
   <211> 20
   <212> DNA
   <213> Human
<400> 96
   aggttagagt gtcgagtagc 20
<210> 97
   <211> 19
   <212> DNA
   <213> Human
<400> 97
   tcgaaaatcg cgtacaccg 19
<210> 98
   <211> 33
   <212> DNA
   <213> Human
<400> 98
   cgcgatcggt gcggttgtga gacggtgatc gcg 33
<210> 99
   <211> 19
   <212> DNA
   <213> Human
<400> 99
   ttcggtaggt tagagtgtc 19
<210> 100
   <211> 20
   <212> DNA
   <213> Human
<400> 100
   ctatcgtctc gaaaatcgcg 20
<210> 101
   <211> 23
   <212> DNA
   <213> Human
<400> 101
   tattttttag gtttcgtttc ggc 23
<210> 102
   <211> 18
   <212> DNA
   <213> Human
<400> 102
   aaacgacccg aatacccg 18
<210> 103
   <211> 32
   <212> DNA
   <213> Human
<400> 103
   cgcgatcgtc ggtcggacgt tcgttgatcg cg 32
<210> 104
   <211> 23
   <212> DNA
   <213> Human
<400> 104
   tattttttag gtttcgtttc ggc 23
<210> 105
   <211> 19
   <212> DNA
   <213> Human
<400> 105
   cgactttcta ctccaaacg 19
<210> 106
   <211> 21
   <212> DNA
   <213> Human
<400> 106
   gtaggtagtt agttggtttt c 21
<210> 107
   <211> 20
   <212> DNA
   <213> Human
<400> 107
   gaaacaaacg acccgaaacg 20
<210> 108
   <211> 36
   <212> DNA
   <213> Human
<400> 108
   cgcgatcgta tttacgggag tcggagtttg atcgcg 36
<210> 109
   <211> 21
   <212> DNA
   <213> Human
<400> 109
   gtaggtagtt agttggtttt c 21
<210> 110
   <211> 18
   <212> DNA
   <213> Human
<400> 110
   gcgaaaattc tctatacg 18
<210> 111
   <211> 21
   <212> DNA
   <213> Human
<400> 111
   tattgtacgg ggttttaagt c 21
<210> 112
   <211> 17
   <212> DNA
   <213> Human
<400> 112
   ttcccttctt tcccacg 17
<210> 113
   <211> 32
   <212> DNA
   <213> Human
<400> 113
   cgcgatcgac gacgggaggg taatggatcg cg 32
<210> 114
   <211> 19
   <212> DNA
   <213> Human
<400> 114
   ggtcgttcgg ttattgtac 19
<210> 115
   <211> 17
   <212> DNA
   <213> Human
<400> 115
   ttcccttctt tcccacg 17
<210> 116
   <211> 18
   <212> DNA
   <213> Human
<400> 116
   cgtggaatag ttgtttgc 18
<210> 117
   <211> 21
   <212> DNA
   <213> Human
<400> 117
   ttaaaacaaa actaaaatac g 21
<210> 118
   <211> 37
   <212> DNA
   <213> Human
<400> 118
   cgcgatcaac ctaatcgaaa cgcaactaaa gatcgcg 37
<210> 119
   <211> 18
   <212> DNA
   <213> Human
<400> 119
   cgtggaatag ttgtttgc 18
<210> 120
   <211> 16
   <212> DNA
   <213> Human
<400> 120
   cgaacctaaa ctcccg 16
<210> 121
   <211> 22
   <212> DNA
   <213> Human
<400> 121
   aggcgatttg agtcgttttt ac 22
<210> 122
   <211> 21
   <212> DNA
   <213> Human
<400> 122
   gacaacctca acaaaaaatc g 21
<210> 123
   <211> 32
   <212> DNA
   <213> Human
<400> 123
   cgcgatcaaa gttgtcggtc gggaggatcg cg 32
<210> 124
   <211> 18
   <212> DNA
   <213> Human
<400> 124
   cgcgttattt cgggaatc 18
<210> 125
   <211> 21
   <212> DNA
   <213> Human
<400> 125
   gacaacctca acaaaaaatc g 21
<210> 126
   <211> 21
   <212> DNA
   <213> Human
<400> 126
   ttcggttttc gcgttttgtt c 21
<210> 127
   <211> 19
   <212> DNA
   <213> Human
<400> 127
   cgaaaactat caaccctcg 19
<210> 128
   <211> 26
   <212> DNA
   <213> Human
<400> 128
   cgcgacggtc gtcgttcggg ttcgcg 26
<210> 129
   <211> 22
   <212> DNA
   <213> Human
<400> 129
   gatataacgt ttttttcgcg tc 22
<210> 130
   <211> 18
   <212> DNA
   <213> Human
<400> 130
   atacccgccc taacgccg 18
<210> 131
   <211> 16
   <212> DNA
   <213> Human
<400> 131
   tcggggtgta gcggtc 16
<210> 132
   <211> 20
   <212> DNA
   <213> Human
<400> 132
   cccaatacta aatcacgacg 20
<210> 133
   <211> 27
   <212> DNA
   <213> Human
<400> 133
   cgcgatgtcg gcgggagttc gatcgcg 27
<210> 134
   <211> 19
   <212> DNA
   <213> Human
<400> 134
   agggcgtttt tttgcggtc 19
<210> 135
   <211> 20
   <212> DNA
   <213> Human
<400> 135
   cccaatacta aatcacgacg 20
<210> 136
   <211> 20
   <212> DNA
   <213> Human
<400> 136
   cgcggtaatt agtatatttc 20
<210> 137
   <211> 20
   <212> DNA
   <213> Human
<400> 137
   gctacgacac tacgcttacg 20
<210> 138
   <211> 25
   <212> DNA
   <213> Human
<400> 138
   cgcgatcggt agttgcgtta tcgcg 25
<210> 139
   <211> 23
   <212> DNA
   <213> Human
<400> 139
   tgttgtggta attagtatat ttt 23
<210> 140
   <211> 23
   <212> DNA
   <213> Human
<400> 140
   caaccactcc aacatacact aca 23
<210> 141
   <211> 25
   <212> DNA
   <213> Human
<400> 141
   cgcgagtagt tgtgttttgt tcgcg 25
<210> 142
   <211> 23
   <212> DNA
   <213> Human
<400> 142
   ggttttaaaa gtyggtgttt att 23
<210> 143
   <211> 21
   <212> DNA
   <213> Human
<220>
   <221> misc_feature
   <222> (3)..(3)
   <223> n is a, c, g, or t
<400> 143
   ccnaacaact acaaccactc c 21

## Description

### Technical field

The present invention relates to detection and treatment of pancreatic cancer.

### Background

Pancreatic cancer is the 4^{th} leading cause of cancer death in the world, even though it only accounts for 2-3 % of all cancer cases. It is one of the most deadly cancers with a 5-year survival rate of less than 10 %. The only chance for cure lies on early detection and complete resection of the tumor. Unfortunately only 10-20 % of the cancers can be curatively resected at the time of diagnosis and despite surgery many patients experience recurrence. The mean survival time of patients, who do not undergo surgery, is 3 to 6 months after the time of diagnosis. One of the main reasons for the high mortality is difficulties in early detection due to lacking or non-specific symptoms during the early stage of the disease. Abdominal pain, weight loss, fatigue and jaundice are very similar to symptoms related to chronic pancreatitis which is an essential differential diagnosis and a known risk factors of pancreatic cancer.

Currently CA-19-9 (Carbohydrate antigen) is the best blood-based marker for pancreatic cancer. CA-19-9 levels can be elevated in pancreatic cancer but frequently only in advanced disease. Elevation of CA-19-9 is also seen in other types of cancer and inflammatory diseases like chronic pancreatitis and especially conditions of benign biliary obstruction. About 10 % of the population lacks the ability to produce CA-19-9, which makes CA-19-9 insufficient as an early diagnostic marker. In the diagnosis of pancreatic cancer advanced imaging modalities, such as PET-CT/three-phase-CT scan, transabdominal ultrasound, endoscopic ultrasound, laparoscopic ultrasound and ERCP are necessary. The drawback is that several of these methods are invasive and entail a risk of complications. Therefore it will be a major advance for the patients if a blood based marker could be used for diagnosis.

Cancer cells releases cell-free DNA into the blood. The DNA fragments have a length of approximately 160 base pairs equivalent with nucleosomal DNA. The cell free DNA can be detected in plasma and serum. The DNA changes are potentially tumor specific and useable in the development of a blood based diagnostic marker for pancreatic cancer.

During the development of pancreatic cancer genetic and epigenetic changes arise. Epigenetic modifications occur at a genomic level, which will not change the sequence of the bases of the DNA. Epigenetic modifications change the DNA conformation, and as a consequence the expression of genes will change. The main epigenetic modifications includes among other DNA hypermethylation, which consists of the addition of a methyl (CH₃) residue on cytosine preceding a guanosine, known as CpG dinucleotides. DNA hypermethylation often occurs in CpG-rich regions (CpG islands) of the promoter sequence of the genes. Hypermethylation in the promoter regions of tumor suppressor genes results in downregulation or silencing of the tumor suppressor function. DNA hypermethylation can be detected in cell-free DNA and the changes are potentially tumor specific and useable in the development of a blood based diagnostic marker for pancreatic cancer.

Cell free DNA hypermethylation as a blood based marker for pancreatic cancer has until now only been studied in few and small studies testing methylation status of only a single gene or small gene panel. Statistically significant difference in DNA hypermethylation between patients with pancreatic cancer and healthy controls has been found. However, it is hard to differentiate between malign and benign pancreatic disease based on changes in hypermethylation status. None of the previous examined genes have the potential to work as an individual diagnostic marker.

The present disclosure provides a broad gene panel, which is capable of determining pancreatic cancer with high sensitivity and specificity.

### Summary

In one aspect, a method is provided of determining pancreatic cancer, a predisposition to pancreatic cancer, the prognosis of a pancreatic, and/or monitoring a pancreatic cancer in a human subject, said method comprising in cell-free DNA from a blood sample from said human subject determining the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

In another aspect, a method is provided for assessing whether a human subject has pancreatic cancer and/or a predisposition to pancreatic cancer, assessing the prognosis of a pancreatic of a pancreatic cancer, and/or monitoring a pancreatic cancer, said method comprising
a) providing a blood sample from said human subject,
b) isolating cell-free DNA from said blood sample,
c) determining in said sample the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM,
d) on the basis of said methylation status identifying a human subject that has pancreatic cancer and/or a predisposition to pancreatic cancer is more likely to develop pancreatic cancer.

### Description of Drawings

Figure 1: Flow diagram of patients included in the study. A) Inclusion of patients with pancreatic adenocarcinoma. B) Inclusion of patients with chronic pancreatitis. C) Inclusion of patients with acute pancreatitis.
Figure 2: Stepwise selection of genes for the pancreatic cancer diagnostic prediction model.
Figure 3: Performance of Model 13 on stage I, II, III and IV pancreatic cancer. Model 13 (age>65, BMP3, RASSF1A, BNC1, MESTv2, TFPI2, APC, SFRP1, SFRP2) AUC= 0·86 (probability cut point of 0·5; sensitivity 75·79% and specificity 83·06%).
Figure 4: Performance of Model 13 on stage I and II pancreatic cancer. Model 13 (age>65, BMP3, RASSF1A, BNC1, MESTv2, TFPI2, APC, SFRP1, SFRP2) AUC= 0·86 (probability cut point of 0·50; sensitivity 72·50% and specificity 83·06%).
Figure 5: Table with descriptive data of the patients in example 1.

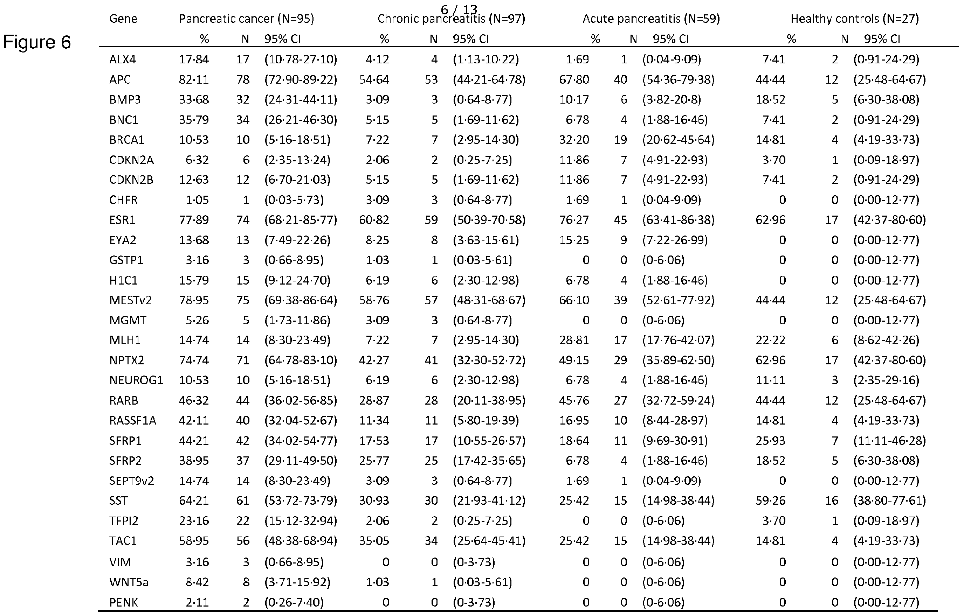

Figure 6: Hypermethylation frequencies for each gene in each group.
Figure 7: Flow diagram of patients included in the study
Figure 8: Stepwise selection of genes for the pancreatic cancer prognostic prediction model (AJCC* stages; I, II, III vs IV).
Figure 9: Performance of the prognostic prediction Model 10; AJCC stage* I, II, III vs IV pancreatic cancer
Figure 10: Stepwise selection of genes for the pancreatic cancer prognostic prediction model (AJCC* stages; I, II vs III, IV).
Figure 11: Performance of the prognostic prediction Model 7; AJCC stage* I, II vs III, IV pancreatic cancer
Figure 12: Genes with statistically significant difference in hypermethylation frequency between AJCC cancer stages
Figure 13: Hypermethylation frequencies for each gene by AJCC staging

### Detailed description

The invention is defined by the claims. Any subject matter falling outside the scope of the claims is provided for information purposes only.

The present invention relates to methylation biomarkers detectable in plasma cell-free DNA for use in the diagnosis and treatment of pancreatic cancer. Generally, the methylation markers of the invention can be used in methods for identifying human subjects, which are predisposed to pancreatic cancer; i.e. subjects having an increased likelihood of developing pancreatic cancer. The methylation markers of the invention can also be used in methods for identifying subjects having pancreatic cancer, and in this case, the markers allow early diagnosis. Further, the markers of the invention provide prognostic information with respect to pancreatic cancer, and thus, the markers can be used to identify a subject having pancreatic cancer, and the cancer DNA can be tested for predictive prognostic information based on the methylation markers of the invention, as well as information on which curative, ameliorative or palliative treatment to provide for the pancreatic cancer. The methylation status of the methylation markers of the invention may also be used to monitor a treatment provided for the curing and/or ameliorating a pancreatic cancer. Additionally, the marker methylation status can be used to monitor relapse of pancreatic cancer for a human subject previously treated for pancreatic cancer.

Thus, aspects of the present invention relates to i) methods for identifying human subjects, which are predisposed to pancreatic cancer, and/or which have a pancreatic cancer, including early stages, such as asymptomatic stages of pancreatic cancer; ii) methods for providing prognostic information of a pancreatic cancer and/, iii) methods of monitoring a treatment of a pancreatic cancer, and/or monitoring relapse of a pancreatic cancer.

In order to facilitate the understanding of the invention a number of definitions are provided below.

### Definitions

Amplification according to the present invention is the process wherein a plurality of exact copies of one or more gene loci or gene portions (template) is synthesised. In one preferred embodiment of the present invention, amplification of a template comprises the process wherein a template is copied by a nucleic acid polymerase or polymerase homologue, for example a DNA polymerase or an RNA polymerase. For example, templates may be amplified using reverse transcription, the polymerase chain reaction (PCR), ligase chain reaction (LCR), in vivo amplification of cloned DNA, isothermal amplification techniques, and other similar procedures capable of generating a complementing nucleic acid sequence.

Amplified copies of a targeted genetic region are sometimes referred to as an amplicon.

A double stranded nucleic acid contains two strands that are complementary in sequence and capable of hybridizing to one another. In general, a gene is defined in terms of its coding strand, but in the context of the present invention, an oligonucleotide primer, which hybridize to a gene as defined by the sequence of its coding strand, also comprise oligonucleotide primers, which hybridize to the complement thereof.

The term "dinucleotide" as used herein refers to two sequential nucleotides. The dinucleotide may be comprised in an oligonucleotide or a nucleic acid sequence. In particular, the dinucleotide CpG, which denotes a cytosine linked to a guanine by a phosphodiester bond, may be comprised in an oligonucleotide according to the present invention, and also comprised in a targeted gene locus sequence according to the present invention. A CpG dinucleotide is also herein referred to as a CpG site. CpG sites are targets for methylation of the cytosine residue.

The gene loci methylation markers of the present invention can be used to infer pancreatic cancer based on the detection of methylation positive cell-free DNA marker loci DNA in a sample comprising in a mixture of cell-free DNA molecules from a subject. For example, the methylation status of a specific gene locus can be detected as methylation positive in those cases where methylation positive DNA can be found in the sample regardless of the relative amount of methylation positive and methylation negative molecules that are present in the sample.

Cell-free DNA is DNA circulating freely in the blood stream.

Pancreatic cancer herein refers to pancreatic adenocarcinoma including any stages thereof, whether symptotic or asymptotic.

The term "predisposition" as used herein in respect of pancreatic cancer is meant to refer to a state of being more likely to develop pancreatic cancer in the future. The term is not meant to imply a genetic disposition as such. However, epigenetic regulation may contribute to predisposition to pancreatic cancer within the meaning of the present disclosure.

### Method of determining pancreatic cancer

The present invention provides a number of methods for analysing a human subject with respect to pancreatic cancer. In particular, the invention provides methods for determining pancreatic cancer in a human subject, methods for determining a predisposition to pancreatic cancer for a human subject, methods for determining the prognosis of a pancreatic cancer and methods for categorizing or staging a pancreatic cancer of a human subject, methods for monitoring a pancreatic cancer, such as monitoring the treatment of a pancreatic cancer and/or relapse of a pancreatic cancer. The methylation biomarkers for pancreatic cancer are described in more detailed herein below. Generally, a group of at least two methylation biomarkers for pancreatic cancer are selected from a gene locus selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

Thus, in one aspect, a method is provided for determining pancreatic cancer, a predisposition to pancreatic cancer, the prognosis of a pancreatic cancer, and/or monitoring a pancreatic cancer in a subject, said method comprising in a sample from said subject determining the methylation status of at least one gene including regulatory sequences of said gene, wherein said gene locus is selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

In another aspect, a method is provided for categorizing or predicting the clinical outcome of a pancreatic cancer in a human subject, said method comprising in a sample from said subject determining the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

In another aspect, a method is provided for evaluating the risk for a human subject of developing pancreatic cancer, or for monitoring relapse of a pancreatic cancer, said method comprising in a sample from said subject determining the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

Furthermore taught is a method for assessing whether a human subject is likely to develop pancreatic cancer, said method comprising
i) providing a blood sample from said human subject,
ii) isolating nucleic acid from said blood sample,
iii) determining in said sample the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM,
iv) on the basis of said methylation status identifying a human subject that is more likely to develop pancreatic cancer.

The methods, inter alia, thus involve determining the methylation status of two or more gene loci as defined herein. Thus, methylation status is determined for multiple gene loci, for example methylation status for at least two gene loci are determined, such as at least three gene loci, such as at least four gene loci, or five or more gene loci. The plurality of gene loci is preferably selected from the gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

**Table 1. Gene names and known function**

| *Gene* | *Name* | *Function* |
|---|---|---|
| *ALX4* | Aristaless-like homeobox 4 | Skull and limb development |
| *APC* | Adenomatous polyposis coli | Tumor suppressor, cellular adhesion, angiogenesis, β-catenin regulation |
| *BMP3* | Bone morphogenetic protein 3 | Bone formation, angiogenesis |
| *BNC1* | Basonuclein 1 | Regulator for rRNA transcription. |
| BRCA1 | Breast cancer 1 | Tumor suppressor, genomic stability, DNA repair |
| *CDKN2B* | Cyclin-dependent kinase inhibitor 2B (P15) | Tumor suppressor, cell-cycle regulation |
| *CHFR* | Checkpoint with forkhead and ring finger domains | Cell-cycle regulation |
| *ESR1* | Estrogen Receptor 1 | Angiogenesis |
| *EYA2* | Transcriptional coactivator and | Eye development, |
| | phosphatase2 | transcription factor |
| *GSTP1* | Glutathione S-transferase pi 1 | Detoxification |
| *HIC1* | Hypermethylated in cancer 1 | Gene transcription and cellular division |
| *MGMT* | O-6-methylguanine-DNA methyltransferase | DNA-repair |
| *MLH1* | MutL homolog 1 | DNA-repair |
| *NEUROG1* | Neurogenin 1 | Neuronal differentiation |
| *MESTv1* | Mesoderm Specific Transcript | Fetal development |
| *NPTX2* | Neural Pentraxin II | Tumor suppressor |
| *p16* | Cyclin-dependent kinase inhibitor 2A | Tumor suppressor, cell-cycle regulation |
| *PENK* | Proenkephalin | NF-κB-pathway (DNA transcription, cell proliferation, apoptosis) |
| *RARB* | Retinoic Acid Receptor Beta | Cell proliferation, cell differentiation |
| *RASSF1A* | Ras association domain family member 1 | Tumor suppressor, cell-cycle regulation and DNA-repair |
| *SEPT9v2* | Septin 9 | Tumor suppressor, cell-cycle regulation, cytokinesis, angiogenesis |
| *SFRP1* | Secreted Frizzled-Related Protein 1 | Angiogenesis, Wnt-pathway (cell proliferation, cell differentiation) |
| *SFRP2* | Secreted frizzled-related protein 2 | Angiogenesis, Wnt-pathway (cell proliferation, cell differentiation) |
| SST | Somatostatin | Cell proliferation |
| *TAC1* | Tachykinin precursor 1 | Neurotransmitting and vasodilation |
| *TFPI2* | Tissue factor pathway inhibitor 2 | Tumor suppressor, angiogenesis |
| *VIM* | Vimentin | Cell-shape and integrity |
| | | maintenance |
| *WNT5a* | Wingless-Type MMTV Integration Site Family, Member 5A | Wnt-pathway (cell proliferation, cell differentiation) |

| | | |
|---|---|---|
| Note. Protein functions are cross-matched with RefSeq: NCBI Reference Sequence Database, http://www.ncbi.nlm.nih.gov/refseq/ | | |

Generally, increased levels of methylation of the respective marker gene loci relative to methylation levels of a predetermined control sample of non-cancer DNA is indicative of the presence of a pancreatic cancer, higher likelihood of developing cancer, decreased overall survival, negative outcome, different stage cancer and/or higher risk of contracting cancer.

Thus, the methods of the invention preferably comprises the steps of comparing the methylation status of the respective gene loci determined for a subject with a predetermined methylation status for the corresponding gene of a reference sample comprising DNA from non-cancer subjects.

The predetermined status is preferably determined from DNA of non-cancer subjects. However, the control group could be patients with pancreatitis, both acute and chronic.

The methods disclosed herein could in principle be applied to any subject whether or not at risk of pancreatic cancer and whether or not suspected of having pancreatic cancer. However, in a preferred embodiment, the methods are applied to subjects, which are suspected of having such cancer or who are at risk of having such cancer based on other symptoms. For example, the methods disclosed herein could preferably be applied to subjects displaying nonspecific symptoms, such as fatigue, jaundice, nausea, loss of appetite and weight loss. The subject can also be an individual for whom an unspecific CT scan signal has been observed in the pancreas, which may or may not be a tumor.

In one embodiment, the methods disclosed herein are applied to a subject having pancreatitis, and the methods disclosed herein are particularly efficient for distinguishing pancreatic cancer from pancreatitis, which may have overlapping symptoms.

### Method for treatment of pancreatic cancer

Aspects of the invention also relates to methods for determining the prognosis of a pancreatic cancer in a human subject and/or inferring a suitable treatment, as well as for monitoring a pancreatic cancer, and in particular monitoring the treatment of a pancreatic cancer and/or monitoring relapse of a pancreatic cancer.

So in one aspect, a method is provided for treatment of pancreatic cancer in a human subject, the method comprises the steps of
i. determining pancreatic cancer, a predisposition to pancreatic cancer, or the prognosis of a pancreatic cancer in a subject by a method of the present invention, as defined elsewhere herein,
ii. selecting human subjects having pancreatic cancer, a predisposition to pancreatic cancer, or a relapse of a pancreatic cancer,
iii. subjecting said subjects identified in step ii. to a suitable treatment for pancreatic cancer.

The step of determining pancreatic cancer by a method of the present invention allows early detection of pancreatic cancer, and therefore allows treatment of the cancer to be initiated before developing into later stages and/or before forming metastases. This increases the possibilities of providing a curative treatment. In a preferred embodiment such treatment is surgical resection of the pancreatic cancer.

If however, surgical resection is not possible, preferred treatments include chemotherapy and/or radiotherapy . A possible treatment is also Irreversible electroporation (IRE), which involves short, repetitive, non-thermal high-energy pulses of electricity to destroy the cancer cells. In one embodiment, the selected human subject is subjected to a treatment selected from surgery, chemotherapy and/or radiotherapy, however, combination thereof can also be applied, such as surgical resection followed by chemotherapy and/or radiotherapy.

The methylation markers also allow monitoring relapse of pancreatic cancer, as well as offering a personalized treatment of pancreatic cancer by surveillance and quality of control of the treatment offered, thereby allowing terminating ineffective treatments and offering alternative treatments. Thus, in another aspect, the teaching provides a method for personalized treatment of a pancreatic cancer of a human subject, said method comprising
i) in a sample from said human subject, determining the methylation status of at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM,
ii) providing a treatment of pancreatic cancer to said human subject,
iii) after a sufficient amount of time having provided the treatment, in a sample from said human subject, determining the methylation status of said at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM,
iv) comparing the methylation status of said gene locus before and after treatment, and v) if methylation of said genetic loci is similar or increased relative to the methylation before treatment, terminating said provided treatment and preferably offering an alternative treatment, or
vi) if methylation of said genetic locus is reduced relative to the methylation before treatment, continuing said provided treatment.

### Methylation biomarkers for pancreatic cancer

As described herein above, the present teaching provides a number of different methods for evaluating pancreatic cancer in a human subject based on methylation status of specific group of gene loci. The teaching also provides specific oligonucleotide primers for use in determining methylation status of specific gene loci, which are methylation biomarkers for pancreatic cancer according to the present teaching. These gene loci include BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

Methylation status is preferably determined in a GC-rich region upstream of the start codon of any gene locus identified herein. Such upstream regions comprise promoter elements and the methylation status is in a most preferred embodiment determined in a promoter region of a marker gene loci identified herein. It is preferably a promoter region located upstream of the open reading frame, i.e. upstream of the start codon (ATG: methionine) or the first exon of the gene encoded by any of the gene loci identified herein, i.e. ALX4, APC, BMP3, BNC1, BRCA1, CDKN2B, CHFR, ESR1, EYA2, GSTP1, HIC1, MGMT, MLH1, NEUROG1, MESTv1, NPTX2, p16, PENK, RARB, RASSF1A, SEPT9v2, SFRP1, SFRP2, SST, TAC1, TFPI2, VIM or WNT5a. The size of a promoter region may vary considerably between genes however a person of skill in the art is able to identify promoter elements on the basis of sequence elements using bioinformatics tools available to him. In the context of the loci identified herein, the promoter region is generally considered to comprise 1000 base pairs upstream of the start codon. Thus in a preferred embodiment, the methylation status is determined in a region within 1000 bp, such as within 900 bp, such as within 800 bp, such as within 700 bp, such as within 600 bp, such as within 500 bp, such as within 400 bp, such as within 300 bp, such as within 200 bp , such as within 100 bp upstream of the start codon of two or more gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM. However, methylation status is in a particular embodiment determined in a region within 1000 bp upstream of the start codon of two or more gene loci selected from any of the subgroups identified herein above, such as
a) BMP3, RASSF1A, BNC1, MESTv2, TFPI2, APC, SFRP1 and SFRP2;
b); MLH1, SEPT9v2, BNC1, ALX4, CDKN2B, NEUROG1, WNT5A and TFPI2;
c) SEPT9v2, SST, ALX4, CDKN2B, HIC1, MLH1, NEUROG1 and BNC1; and
d) ALX4, BNC1, HIC1, Sept9v2, SST, TFPI2 and TAC1.

Generally, in the methods taught, the methylation status is determined for at least two gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

In one embodiment, the methylation status is determined for at least two gene loci selected from the group consisting BMP3, RASSF1A, BNC1, MESTv2, TFPI2, APC, SFRP1 and SFRP2. For example the methylation status is determined at 3, 4, 5, 6, 7 or preferably 8 gene loci of the group consisting BMP3, RASSF1A, BNC1, MESTv2,TFPI2, APC, SFRP1 and SFRP2. Thus, in a preferred embodiment, the methylation status is determined in BMP3, RASSF1A, BNC1, MESTv2, TFPI2, APC, SFRP1 and SFRP2.

In one embodiment, methylation status is determined for at least BMP3, RASSF1A and BNC1. In another embodiment, methylation status is determined for at least BMP3, RASSF1A, BNC1 and MESTv1. In another embodiment, methylation status is determined for at least BMP3, RASSF1A, BNC1, MESTv1 and TFPI2. In another embodiment, methylation status is determined for at least BMP3, RASSF1A, BNC1, MESTv1, TFPI2 and APC. In another embodiment, methylation status is determined for at least BMP3, RASSF1A, BNC1, MESTv1, TFPI2, APC and SFRP1. In another embodiment, methylation status is determined for at least BMP3, RASSF1A, BNC1, MESTv1, TFPI2, APC, SFRP1 and SFRP2. In another embodiment, methylation status is determined for at least BMP3, RASSF1A, BNC1, MESTv1, TFPI2, APC, SFRP1, SFRP2 and EYA2. In another embodiment, methylation status is determined for at least BMP3, RASSF1A, BNC1, MESTv1, TFPI2, APC, SFRP1, SFRP2, EYA2 and NPTX2. In another embodiment, methylation status is determined for at least BMP3, RASSF1A, BNC1, MESTv1, TFPI2, APC, SFRP1, SFRP2, EYA2, NPTX2 and SEPT9v2. In another embodiment, methylation status is determined for at least BMP3, RASSF1A, BNC1, MESTv1, TFPI2, APC, SFRP1, SFRP2, EYA2, NPTX2, SEPT9v2 and WNT5a. In another embodiment, methylation status is determined for at least BMP3, RASSF1A, BNC1, MESTv1, TFPI2, APC, SFRP1, SFRP2, EYA2, NPTX2, SEPT9v2, WNT5a and CDKN2B. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B and ALX4. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4 and HIC1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1 and RARB. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB and SST. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST and ESR1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1 and TAC1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1 and BRCA1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1 and CHFR. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR and GSTP1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1 and MGMT. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT and MLH1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1 and NEUROG1. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1 and p16. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16 and PENK. In another embodiment, methylation status is determined for at least BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16 PENK and VIM.

In another embodiment, the methylation status is determined for at least two gene loci selected from the group consisting of MLH1, SEPT9v2, BNC1, ALX4, CDKN2B, NEUROG1, WNT5A and TFPI2. For example, the methylation status is determined at 3, 4, 5, 6, 7 or preferably 8 gene loci of the group consisting MLH1, SEPT9v2, BNC1, ALX4, CDKN2B, NEUROG1, WNT5A and TFPI2. Thus, in a preferred embodiment, the methylation status is determined in MLH1, SEPT9v2, BNC1, ALX4, CDKN2B, NEUROG1, WNT5A and TFPI2.

In another embodiment, the methylation status is determined for at least two gene loci selected from the group consisting of ALX4, BNC1, HIC1, Sept9v2, SST, TFPI2 and TAC1. For example, the methylation status is determined at 3, 4, 5, 6 or preferably 7 gene loci of the group consisting ALX4, BNC1, HIC1, Sept9v2, SST, TFPI2 and TAC1. Thus, in a preferred embodiment, the methylation status is determined in ALX4, BNC1, HIC1, Sept9v2, SST, TFPI2 and TAC1.

In another embodiment, the methylation status is determined for at least two gene loci selected from the group consisting of SEPT9v2, SST, ALX4, CDKN2B, HIC1, MLH1, NEUROG1 and BNC1. For example, the methylation status is determined at 3, 4, 5, 6, 7 or preferably 8 gene loci of the group consisting SEPT9v2, SST, ALX4, CDKN2B, HIC1, MLH1, NEUROG1 and BNC1. Thus, in a preferred embodiment, the methylation status is determined in SEPT9v2, SST, ALX4, CDKN2B, HIC1, MLH1, NEUROG1 and BNC1.

In one embodiment, the methylation status is determined for the gene loci BNC1 and TFPI2.

In another embodiment, the methylation status is determined for the gene loci ALX4, HIC1, Sept9v2 and SST. In another embodiment, the methylation status is determined for the gene loci ALX4 and SST. In another embodiment, the methylation status is determined for the gene loci ALX4, HIC1 and SST. In another embodiment, the methylation status is determined for the gene loci ALX4 and HIC1. In another embodiment, the methylation status is determined for the gene loci ALX4, Sept9v2 and SST. In another embodiment, the methylation status is determined for the gene loci HIC1, Sept9v2 and SST. In another embodiment, the methylation status is determined for the gene loci ALX4, HIC1, Sept9v2 and SST.

In a preferred embodiment, the methylation status is determined by a method comprising amplifying a gene locus of the invention. In a preferred embodiment, amplification is conducted using at least one primer selected from table 1 which identify specific primers and probes for each gene locus. For example for SST, using SEQ ID NO: 1 and 2 and/or 3 and 4. Thus, it is preferred that at least one primer is selected from the group consisting of SEQ ID Nos: 1-135

### Sample

According to the present invention, the methylation status of one or more gene loci is determined in a blood sample from a human subject. The sample can also be a plasma sample, or a plasma sample can be prepared from the blood sample by centrifugation. The sample is preferably a cell-free DNA sample. The sample of the invention comprises biological material in the form of nucleic acid molecules. The nucleic acid molecules should be extracted from the sample prior to the analysis.

The nucleic acid to be analysed for the presence of methylated CpG may be extracted from the samples by a variety of techniques such as that described by Maniatis, et al (Molecular Cloning: A Laboratory Manual, Cold Spring Harbor, N.Y., pp 280, 281, 1982).

Any nucleic acid, in purified or nonpurified form, can be utilized as the starting nucleic acid or acids, provided it contains, or is suspected of containing, the specific nucleic acid sequence containing the methylation target site (e.g., CpG). The specific nucleic acid sequence which is to be amplified may be a part of a larger molecule or is present initially as a discrete molecule. The nucleic acid sequence to be amplified need not to be present in a pure form, it may for example be a fraction of a complex mixture of other DNA molecules, and/or RNA. In one example, the nucleic acid sequence is a fraction of a genomic nucleic acid preparation.

Extremely low amounts of nucleic acid may be used as target sequence according to the methods of the present invention. It is appreciated by the person skilled in the art that in practical terms no upper limit for the amount of nucleic acid to be analysed exists. The problem that the skilled person may encounter is that the amount of nucleic acid to be analysed is limited. Therefore, it is beneficial that the method of the present invention can be performed on small amounts of nucleic acids. The present methods allow the detection of very few nucleic acid copies. The amount of the nucleic acid to be analysed is in one embodiment at least 0.01 ng, such as 0.1 ng, such as 0.5 ng, for example 1 ng, such as at least 10 ng, for example at least 25 ng, such as at least 50 ng, for example at least 75 ng, such as at least 100 ng, for example at least 125 ng, such as at least 150 ng, for example at least 200 ng, such as at least 225 ng, for example at least 250 ng, such as at least 275 ng, for example at least 300 ng, 400 ng, for example at least 500 ng, such as at least 600 ng, for example at least 700 ng, such as at least 800, ng, for example at least 900 ng or such as at least 1000 ng.

### Methylation status

The methods taught for determining pancreatic cancer in a human subject, methods for determining a predisposition to pancreatic cancer for a human subject, methods for determining the prognosis of a pancreatic cancer in a subject methods for categorizing or staging a pancreatic cancer of a human subject and methods for monitoring a pancreatic cancer, all include a step of providing or obtaining a sample from the human subject, and in that sample determining the methylation status of at least two genetic loci selected from the group consisting of of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM., as well as subregions thereof, in particular promoter regions, such as the regions within 1000 bp upstream of the start codon, including the subregions delineated by the respective primer pairs identified in table 2, such as primer pairs 1 and 2, 3 and 4, 6 and 7, 8 and 9, 11 and 12 etc.

Methylation status of the target gene loci or genetic regions of the present invention may be determined by any suitable method available to the skilled person for detecting methylation status. In one embodiment, methylation status is determined by a quantitative method, which is capable of detecting levels of methylation positive alleles and/or methylation negative alleles in a population of target molecules present in a sample. For example, the quantitative method is preferably capable of detecting different levels of methylation positive alleles of a given target locus sequence, such as detecting whether 0%, less than 1%, more that 1%, such as approximately 10%, 25%, 50%, 75% or 100% of the alleles of a given marker locus are methylation positive. Some techniques in the art detect the presence of one or more methylation positive and/or methylation negative alleles of a given target sequence without providing quantitative data, and without providing information of the relative levels of methylation positive and methylation negative alleles.

Methylation status: the term "methylation status" as used herein, refers to the presence or absence of methylation in a specific nucleic acid region. In particular, the present invention relates to detection of methylated cytosine (5-methylcytosine). A nucleic acid sequence, e.g. a gene locus of the invention, may comprise one or more CpG methylation sites. The nucleic acid sequence of the gene locus may be methylated on all methylation sites (i.e. 100% methylated) or unmethylated on all methylation sites (i.e. 0% methylated). However, the nucleic acid sequence may also be methylated on a subset of its potential methylation sites (CpG-sites). In this latter case, the nucleic acid molecule is heterogeneously methylated. In a preferred embodiment, methylation status refers to the presence or absence of methylated nucleic acid of two or more of the loci described herein, preferably promoter regions of the loci described herein.

The methods for inferring pancreatic cancer of the present invention thus include determining methylation status of specific methylation markers by determining whether a specific methylation marker in a sample obtained or provided from a subject is methylation positive or methylation negative as well as detecting the relative level of methylated alleles of a given locus.

In a preferred embodiment, the methylation status is determined by methylation specific PCR, preferably on cell-free DNA, where the DNA is modified by an agent capable of converting unmethylated cytosine residues.

In another embodiment, the methylation status is determined by use of methylation-sensitive restriction enzymes. Many restriction enzymes are sensitive to the DNA methylation states. Cleavage can be blocked or impaired when a particular base in the recognition site is modified. For example, the MspJI family of restriction enzymes has been found to be dependent on methylation and hydroxymethylation for cleavage to occur. These enzymes excise ~ 32 base pair fragments containing a centrally located 5-hmC or 5-mC modified residue that can be extracted and sequenced. Due to the known position of this epigenetic modification, bisulfite conversion is not required prior to downstream analysis.

Methylation-sensitive enzymes are well-known in the art and include:
Aatll, Accll, Aor13HI, Aor51HI, BspT104I, BssHII, Cfr10I, Clal Cpol, Eco52I, Haell, Hapll, Hhal, Mlul, Nael, Notl, Nrul, Nsbl, PmaCI, Psp1406I, Pvul, Sacll, SaII, Smal and SnaBI.

The digested nucleic acid sample is subsequently analysed by for example gel electrophoresis.

So, in one embodiment of the methods of the underlying teachings, methylation status is determined by a method comprising the steps of
i) providing a blood sample from a human subject,
ii) isolating and processing nucleic acid comprised in said sample using one or more methylation-sensitive restriction endonuclease enzymes,
iii) optionally, amplifying said processed nucleic acid sequence in order to obtain an amplification product, and
iv) analyzing said processed nucleic acid sequence or said amplification product for the presence of processed and/or unprocessed nucleic acid sequences, thereby inferring the presence of methylated and/or unmethylated nucleic acid sequences, wherein methylation status is determined for at least two gene loci selected from BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM.

Methylation status can be determined by modifying the DNA with an agent which targets either methylated or unmethylated sequences, amplifying the DNA, and analysing the amplification products.

For example, amplification product is analysed by detecting the presence or absence of amplification product, wherein the presence of amplification product indicates that the target nucleic acid has not been cleaved by the restriction enzymes, and wherein the absence of amplification product indicates that the target nucleic acid has been cleaved by the restriction enzymes.

Thus, generally, the in the methods of the invention methylation status is determined by a method comprising the steps of
i) providing a blood sample from said subject comprising nucleic acid material,
ii) optionally, isolating nucleic acid material from said blood sample,
iii) modifying said nucleic acid material using an agent, which modifies nucleic acid sequences in a methylation-dependent manner,
iv) amplifying at least one portion of said gene locus using primers, which span or comprise at least one CpG dinucleotide in said gene locus in order to obtain an amplification product, and
v) analyzing said amplification product for the presence of modified and/or unmodified cytosine residues, wherein the presence of modified cytosine residues are indicative of methylated cytosine residues.

For example, the method comprises the steps of
i) providing a sample, such as a blood sample, from said subject comprising nucleic acid material comprising said gene locus,
ii) optionally, isolating nucleic acid material from said blood sample,
iii) modifying said nucleic acid material using an agent, which modifies nucleic acid sequences in a methylation-dependent manner,
iv) amplifying at least one portion of said gene locus using primers, which span or comprise at least one CpG dinucleotide in said gene locus in order to obtain an amplification product, and
v) analyzing said amplification product.

The amplification product can be analysed for nucleic acid substitutions resulting from conversion of modified cytosine residues, preferably wherein the presence of converted cytosine residues are indicative of unmethylated cytosine residues, and presence of unconverted cytosine residues is indicative of methylated cytosine residues. Typically, unmethylated cytosine is converted to thymidine after bisulphite treatment and amplification, while methylated cytosine is left unchanged after same treatment.

In a preferred embodiment, the nucleic acid material is modified using bisulfite and amplified using primers, which span at least one CpG dinucleotide in said gene loci. In this case, amplification primers specific for modified nucleic acid will only support amplification and generate an amplification product, for nucleic acids, which are unmethylated. In contrast, amplification primers specific for unmodified nucleic acid will only support amplification and generate an amplification product, for nucleic acids, which are methylated.

The amplification product, the amplicon, is in a preferred embodiment a genetic region of a gene of the teachings, wherein said region is delineated by the primer pairs identified in table 2.

### Modification of DNA

The method for determining methylation status in the present invention preferably comprise a step of modifying the nucleic acids comprised in the sample, or extracted from the sample, using an agent which specifically modifies unmethylated cytosine in the nucleic acid. As used herein the term "modifies" refers to the specific modification of either an unmethylated cytosine or a methylated cytosine, for example the specific conversion of an unmethylated cytosine to another nucleotide which will distinguish the modified unmethylated cytosine from a methylated cytosine. In one preferred embodiment, an agent modifies unmethylated cytosine to uracil. Such an agent may be any agent capable of said conversion, wherein unmethylated cytosine is modified, but not methylated cytosine. In one preferred embodiment the agent for modifying unmethylated cytosine is sodium bisulfite. Sodium bisulfite (NaHSO₃) reacts readily with the 5,6-double bond of cytosine, but only poorly with methylated cytosine. The cytosine reacts with the bisulfite ion, forming a reaction intermediate in the form of a sulfonated cytosine which is prone to deamination, eventually resulting in a sulfonated uracil. Uracil can subsequently be formed under alkaline conditions which removes the sulfonate group.

During a nucleic acid amplification process, uracil will by the Taq polymerase be recognised as a thymidine. The product upon PCR amplification of a Sodium bisulfite modified nucleic acid contains cytosine at the position where a methylated cytosine (5-methylcytosine) occurred in the starting template DNA of the sample. Moreover, the product upon PCR amplification of a Sodium bisulfite modified nucleic acid contains thymidine at the position where an unmethylated cytosine (5-methylcytosine) occurred in the starting template DNA of the sample. Thus, an unmethylated cytosine is converted into a thymidine residue upon amplification of a bisulfite modified nucleic acid.

In a preferred embodiment of the present invention, the nucleic acids are modified using an agent which modifies unmethylated cytosine in the nucleic acid. In a specific embodiment, such an agent is a bisulfite, hydrogen sulfite, and/or disulfite reagent, for example sodium bisulfite.

However, in another embodiment, an agent is used, which specifically modifies methylated cytosine in the nucleic acid and does not modify unmethylated cytosine.

### Amplifying step

After modification of the nucleic acids of the sample, the specific genetic region selected for determination of methylation status is preferably amplified in order to generate and thereby obtain multiple copies (amplicons) of the respective genetic regions, which can allow its further analysis with respect to methylation status. The amplification is preferably performed using at least one oligonucleotide primer, which targets the specific genetic region comprising methylation markers for pancreatic cancer according to the present invention. Most preferably amplification is performed using two oligonucleotide primers, which delineates the analysed region. The skilled person may use his common general knowledge in designing suitable primers. However, in a preferred embodiment, at least one, and preferably two methylation-independent oligonucleotide primers are employed for amplification of the modified nucleic acid. The nature of methylation-independent primers is described on more detail herein below.

The amplifying step is a polymerisation reaction wherein an agent for polymerisation is involved, effecting an oligonucleotide primer extension. The agent for polymerization may be any compound or system which will function to accomplish the synthesis of primer extension products, including enzymes. Enzymes that are suitable for this purpose include, for example, E. coli DNA polymerase I, Klenow fragment of E. coli DNA polymerase I, T4 DNA polymerase, other available DNA polymerases, polymerase muteins, reverse transcriptase, and other enzymes, including heat-stable enzymes (i.e., those enzymes which perform primer extension after being subjected to temperatures sufficiently elevated to cause denaturation also known as Taq polymerases). Suitable enzymes will facilitate combination of the nucleotides in the proper manner to form the primer extension products which are complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

The amplification product (amplicon) may be of any length, however usually, the amplification product comprise between 15 and 1000 nucleotides, such as between 15 and 500 nucleotides, such as between 50 and 120 nucleotides, preferably between 80 and 100 nucleotides. In a preferred embodiment, the amplicon is delineated by the primers identified in table 2 for each respective gene, cf. herein above.

The PCR reaction is characterised by three steps a) melting a nucleic acid template, b) annealing at least one methylation-independent oligonucleotide primer to said nucleic acid template, and c) elongating said at least one methylation-independent oligonucleotide primer. These three steps are repeated through multiple cycles, as is well known to those of skill in the art.

PCR is usually performed on a PCR machine, which is also known as a thermal cycler. Specifically, the thermal cycler may be coupled to a fluorometer, thus allowing the monitoring of the nucleic acid amplification in real time by use of intercalating fluorescent dyes, or other fluorescent probes. Applicable dyes according to the present invention include any DNA intercalating dye. Examples of methylation specific probes are listed in table 2 in respect of each gene locus provided herein, e.g. SEQ ID NO: 5 for detection of an amplicon of the SST gene, SEQ ID NO: 8 for detection of the APC gene etc.

Suitable dyes include ethidium bromide, EvaGreen, LC Green, Syto9, SYBR Green, SensiMix HRM^{™} kit dye, however many dies are available for this same purpose.

Real-time PCR allows for easy performance of quantitative PCR (qPCR), which is usually aided by algorithms comprised in the software, which is usually supplied with the PCR machines.

The fluorometer can furthermore be equipped with software that will allow interpretation of the results. Such software for data analyses may also be supplied with the kit of the present invention.

Another variant of the PCR technique, multiplex PCR, enables the simultaneous amplification of many targets of interest in one reaction by using more than one pair of primers.

PCR according to the present invention comprise all known variants of the PCR technique known to people of skill within the art. Thus, the PCR technology comprise real-time PCR, qPCR, multiplex PCR.

### Oligonucleotide primers

The oligonucleotide primers of the present invention are capable of being employed in amplification reactions, wherein the primers are used in amplification of template DNA originating from a methylation-positive or methylation-negative strand. The preferred primers of the present invention comprise at least one CpG dinucleotide.

The design of oligonucleotide primers suitable for nucleic acid amplification techniques, such as PCR, is known to people skilled within the art. The design of such primers involves analysis of the primer's melting temperatures and ability to form duplexes, hairpins or other secondary structures. Both the sequence and the length of the oligonucleotide primers are relevant in this context. The oligonucleotide primers according to the present invention comprise between 10 and 200 consecutive nucleotides, such as at least 60, at least 65, at least 70, at least 75, at least 80, at least 85, at least 90, at least 95, at least 100, at least 110, at least 120, at least 130, at least 140, at least 150, at least 160, at least 180 or at least 200 nucleotides. In a specific embodiment, the oligonucleotide primers comprise between 15 and 60 consecutive nucleotides, such as 15, 16, 17, 18, 19, 20, preferably 21, 22, 23, 24, 25, 26, 27, 28, 29, 30, such as 31, 32, 33, 34, 35, 36, 37, 38, 39, 40, alternatively at least 41, at least 42, at least 44, at least 46, at least 48, at least 50, at least 52, at least 54, at least 56, at least 58, or at least 60 consecutive nucleotides.

The methods employed for determining the methylation status of a nucleic acid according to the present invention, preferably comprise amplification of a modified nucleic acid by use of a methylation independent oligonucleotide primer. In one embodiment, the oligonucleotide primers of the present invention are able to hybridize to a nucleic acid sequence comprising CpG islands. In one embodiment, the oligonucleotide primers comprise 2, alternatively 3, 4, 5, 6, 7, 8, 9 or 10 CpG dinucleotides. Primers specific for methylated alleles recognize the CpG dinucleotides present in the target nucleic sequence, whereas primers specific for unmethylated alleles are designed to recognize TpG dinucleotides due the PCR mediated conversion of unmethylated C residues to thymine.

In one embodiment, the oligonucleotide primer of the present invention is selected from the group of primers identified in table 2. Methylation status is preferably determined for a gene mentioned in table 2 using the respective forward primer and reverse primer set out in table 2.

In one embodiment, the oligonucleotide primers hybridize to a target nucleic acid sequence of a gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM, or the complement thereof.

In one embodiment, an oligonucleotide primer of the present teachings specifically hybridizes to regions within 1000 bp upstream of the start codon of gene loci selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM, or the complement thereof.

In another embodiment, the oligonucleotide primer hybridizes to a target nucleic acid sequence of a gene locus selected from the group consisting of BMP3, RASSF1A, BNC1, MESTv2, TFPI2, APC, SFRP1 and SFRP2, or the complement thereof, preferably to a target sequence within 1000 bp upstream of the start codon.

In another embodiment, the oligonucleotide primer hybridizes to a target nucleic acid sequence of a gene locus selected from the group consisting of MLH1, SEPT9v2, BNC1, ALX4, CDKN2B, NEUROG1, WNT5A and TFPI2, or the complement thereof, preferably to a target sequence within 1000 bp upstream of the start codon.

In another embodiment of the present teachings the oligonucleotide primer hybridizes to a target nucleic acid sequence of a gene locus selected from the group consisting of ALX4, BNC1, HIC1, Sept9v2, SST, TFPI2 and TAC1, or the complement thereof, preferably to a target sequence within 1000 bp upstream of the start codon.

In another embodiment of the present teachings the oligonucleotide primer hybridizes to a target nucleic acid sequence of a gene locus selected from the group consisting of SEPT9v2, SST, ALX4, CDKN2B, HIC1, MLH1, NEUROG1 and BNC1, or the or the complement thereof, preferably to a target sequence within 1000 bp upstream of the start codon.

In one embodiment, an oligonucleotide primer of the present invention specifically comprises or consists of 5-50, such as 5-30, such as 10-20 consecutive nucleotides of a subsequence (preferably promoter region subsequence) of a gene locus selected from the group consisting of BMP3, RASSF1A, BNC1, MESTv1, TFPI2, APC, SFRP1, SFRP2, EYA2, NPTX2, SEPT9v2, WNT5a, CDKN2B, ALX4, HIC1, RARB, SST, ESR1, TAC1, BRCA1, CHFR, GSTP1, MGMT, MLH1, NEUROG1, p16, PENK and VIM, or the complement thereof.

### Analysis of amplified CpG-containing nucleic acids

According to the present invention the nucleic acid (target) sample is subjected to an agent that converts an unmethylated cytosine to another nucleotide which will distinguish the unmethylated from the methylated cytosine. In a preferred embodiment the agent modifies unmethylated cytosine to uracil. The modifying agent can be sodium bisulphite. During the amplification process uracil will be converted to thymidine.

Thus, after conversion of unmethylated cytosines to uracils in the nucleic acid (target) sample, the subsequent PCR amplification converts uracils to thymine. As a consequence of the sodium bisulfite and PCR-mediated specific conversion of unmethylated cytosines to thymines, G:C base pairs are converted to A:T base pairs at positions, where the cytosine was methylated.

The difference in nucleic acid sequence at previously methylated (methylation positive) or unmethylated (methylation negative) cytosines allows for the analysis of methylation status in a sample. This analysis can comprise identifying cytosine residues, which have been converted to thymidine after amplification, as unmethylated cytosine residues, and identifying cytosine residues, which has not been converted, as methylated cytosine residues.

By this method, analysis of the amplified nucleic acid after treatment with a modifying agent such as sodium bisulphite and subsequent PCR amplification can reveal the methylation status of the target nucleic acid sequence. Thus, in one embodiment, the method for determining methylation status of a nucleic acid according to the present invention further comprises a step of analyzing the amplified nucleic acids.

Specifically, the subsequent analysis can be selected from the group consisting of melting curve analysis, high resolution melting analysis, nucleic acid sequencing, primer extension, denaturing gradient gel electrophoresis, southern blotting, restriction enzyme digestion, methylation-sensitive single-strand conformation analysis (MS-SSCA) and denaturing high performance liquid chromatography (DHPLC).

In one embodiment, the methylation status of the amplified nucleic acid is determined by any method selected from the group consisting of Methylation-Specific PCR (MSP), Whole genome bisulfite sequencing (BS-Seq), HELP assays, ChIP-on-chip assays, Restriction landmark genomic scanning, Methylated DNA immunoprecipitation (MeDIP), Pyrosequencing of bisulfite treated DNA, Molecular break light assays, and Methyl Sensitive Southern Blotting.

In a preferred embodiment, the methylation status of the amplified nucleic acid (amplification product/amplicon) is determined by Methylation-Specific PCR (MSP), wherein the primers sequence determines whether an amplicon is generated, because one set of primers only support amplification of methylated alleles and another set of primers only support amplification of unmethylated alleles. In this case, analysis of the amplicon involves detection of the relative amount of each group of amplicons, methylation-specific amplicons vs. unmethylation specific amplicons.

In another embodiment, the methylation status of the amplified containing nucleic acid is determined by a method selected from the group consisting bisulfite sequencing, COBRA, melting curve analysis, or DNA methylation arrays.

In one embodiment, the analysis of the amplified nucleic acid region is melting curve analysis. In another embodiment, the analysis of the amplified nucleic acid is high resolution melting analysis (HRM).

### Sequences

**Table 2. DNA sequences for probes and primers**

| SEQ ID NO | Gene | DNA sequence | Amplicon size |
|---|---|---|---|
| 1 | SST M1 | GCG TCG AGA TGT TGT TTT GTC | |
| 2 | SST M2 | CCA AAA CCA AAA CGA TAA ACA ACG | 67 |
| 3 | SST M beacon | (HEX)CGA TCG ACC AAC +GC+G CAC TAA CGA TCG(Dabcyl) | |
| 4 | SST Am | AGA GTA TAT AAG TCG TTT TAG GAG C | |
| 5 | SST Bm | CCA AAA CCA AAA CGA TAA ACA ACG | 83 |
| 6 | APC M1 | AGT GCG GGT CGG GAA GC | |
| 7 | APC M2 | AAT CGA CGA ACT CCC GAC G | 93 |
| 8 | APC M beacon | (HEX)CGC GAT CGT TG+G ATG +CG+G AAT CGC G(Dabcyl) | |
| 9 | APC Am | ATT GCG GAG TGC GGG TC | |
| 10 | APC Bm | AAT CGA CGA ACT CCC GAC G | 99 |
| 11 | MLH1 M1 | TGG TTT TTT GGC GTT AAA ATG TC | |
| 12 | MLH1 M2 | AAA TAA CTT CCC CCG CCG | 100 |
| 13 | MLH1 M beacon | (HEX)CGC GAT CTC +GTC CAA CC+G CC+G AAT ATC GCG(Dabcyl) | |
| 14 | MLH1 Am | TGG TTT TTT GGC GTT AAA ATG TC | |
| 15 | MLH1 Bm | CAT CTC TTT AAT AAC ATT AAC TAA CCG | 124 |
| 16 | SFRP1 M1 | GGA GTT GAT TGG TGG TTG CGC | |
| 17 | SFRP1 M2 | CGC GAC ACT AAC TCC G | 90 |
| 18 | SFRP1 M beacon | (HEX)CGC GAT G+GT T+CG +GTC G+TA ATC GCG(Dabcyl) | |
| 19 | SFRP1 Am | GAG GCG ATT GGT TTT CGC | |
| 20 | SFRP1 Bm | CGC GAC ACT AAC TCC G | 121 |
| 21 | CHFR M1 | GTT TCG GTT TTA GTT TCG TAT TTC | |
| 22 | CHFR M2 | CGA CTC CTA CGT CTA AAC GCG | 102 |
| 23 | CHFR M beacon | (HEX)CGC GAT CCG +CA+C GT+C CAT CGC G(Dabcyl) | |
| 24 | CHFR Am | GTT TCG GTT TTA GTT TCG TAT TTC | |
| 25 | CHFR Bm | CCC TAA AAA CGA CTC CTA CG | 111 |
| 26 | RASSF1A M1 | GGG AGG CGT TGA AGT C | |
| 27 | RASSF1A M2 | GTA CTT CGC TAA CTT TAA ACG | 76 |
| 28 | RASSF1A M beacon | (HEX)CGC GAT TCG +TT+C G+GT TCG CTC GCG(Dabcyl) | |
| 29 | RASSF1A Am | GGG AGG CGT TGA AGT C | |
| 30 | RASSF1A Bm | A ATA AAC TCA AAC TCC CCC G | 115 |
| 31 | CDKN2A M1 | TTT CGA GTA TTC GTT TAT AGC | |
| 32 | CDKN2A M2 | TTT CTT CCT CCG ATA CTA ACG | 113 |
| 33 | CDKN2A M beacon | (HEX)CGA CGT G+AA +AGA +TAT CG+C G+GT ACT TCG(Dabcyl) | |
| 34 | CDKN2A Am | TGT TCG GAG TTA ATA GTA TTT TTT TC | |
| 35 | CDKN2A Bm | TTT CTT CCT CCG ATA CTA ACG | 134 |
| 36 | RARB M1 | GGG TAT CGT CGG GGT AGA TTC | |
| 37 | RARB M2 | TCG ACC AAT CCA ACC GAA ACG | 113 |
| 38 | RARB M beacon | (HEX)CGC GAC GAA +TA+C GTT +CCG AAT CGC G(Dabcyl) | |
| 39 | RARB Am | AGT AGG GTT TGT TTG GGT ATC | |
| 40 | RARB Bm | TCG ACC AAT CCA ACC GAA ACG | 127 |
| 41 | ESR1 M1 | GGG ATT GTA TTT GTT TTC GTC | |
| 42 | ESR1 M2 | ACG CAA CGC ATA TCC CG | 104 |
| 43 | ESR1 M beacon | (HEX)CGC GAT GAA +CGA +CCC G+AC GAT CGC G(Dabcyl) | |
| 44 | ESR1 Am | GTT TTG GGA TTG TAT TTG TTT TC | |
| 45 | ESR1 Bm | ACG CAA CGC ATA TCC CG | 109 |
| 46 | BRCA1 M1 | TCG TGG TAA CGG AAA AGC GCG | |
| 47 | BRCA1 M2 | CCG TCC AAA AAA TCT CAA CG | 85 |
| 48 | BRCA1 M beacon | (HEX)CGA TCG G+CG GCG+TG+A GCG TAC G(Dabcyl) | |
| 49 | BRCA1 Am | | |
| 50 | BRCA1 Bm | | 114 |
| 51 | MESTv2 M1 | CGA CGT TTT AGT TTC GAG TC | |
| 52 | MESTv2 M2 | CGC TTC CAT AAA CCA AAA ATT CTCG | 87 |
| 53 | MESTv2 M beacon | (HEX)CGA TCG G+TG +GT+C G+GG TTC GAT CG(Dabcyl) | |
| 54 | MESTv2 Am | GCG ATG GGT TTG TGC GC | |
| 55 | MESTv2 Bm | GAA AAA CCG ATT ACG CAT ACG | 130 |
| 56 | MGMT M1 | GAT ATG TTG GGA TAG TTC GC | |
| 57 | MGMT M2 | GCA CTC TTC CGA AAA CGA AAC G | 111 |
| 58 | MGMT M beacon | (HEX)CGC GAT CG+T ATC G+TT +TG+C GAT +TTA TCG CG(Dabcyl) | |
| 59 | MGMTAm | GAT ATG TTG GGA TAG TTC GC | |
| 60 | MGMT Bm | AAA AAA CTC CGC ACT TCCG | 129 |
| 61 | SEPT9v2 M1 | GTT TAG TAT TTA TTT TCG AAG TTC | |
| 62 | SEPT9v2 M2 | CCT CCG CGC GAC CCG | 85 |
| 63 | SEPT9v2 M beacon | (FAM)CGA CGT ATT TAG TTG CGC GTT GAT CGA CGT CG(Dabcyl) | |
| 64 | SEPT9v2 Am | GTT TAG TAT TTA TTT TCG AAG TTC | |
| 65 | SEPT9v2 Bm | GCC GAA AAC GCT TCC TCG | 124 |
| 66 | VIM M1 | ATA TTT ATC GCG TTT TCG TTC | |
| 67 | VIM M2 | ACG AAC CTA ATA AAC ATA ACT ACG | 102 |
| 68 | VIM M beacon | (FAM)CGA CGT GTT CGC GTT ATC GTC GTC GAC GTC G(Dabcyl) | |
| 69 | VIM Am | GAG GTT TTC GCG TTA GAG AC | |
| 70 | VIM Bm | ACG AAC CTA ATA AAC ATA ACT ACG | 143 |
| 71 | EYA2 M1 | CGG AGG TAG CGG TAA C | |
| 72 | EYA2 M2 | CGA TAC GAA CGA ACG AAC G | 93 |
| 73 | EYA2 M beacon | (FAM)CGA CGT GTT CGC GTT ATC GTC GTC GAC GTC G(Dabcyl) | |
| 74 | EYA2 Am | GGG TCG GTT TTT TCG GC | |
| 75 | EYA2 Bm | ACG AAT CCC GAC GAA CG | 104 |
| 76 | BMP3 M1 | AGT GGA GAC GGC GTT C | |
| 77 | BMP3 M2 | CTT ACT ACG CTA ACC CAA CG | 96 |
| 78 | BMP3 M beacon | (FAM)CGT CGA GCG GGT GAG GTT CGC GTA TCG ACG(Dabcyl) | |
| 79 | BMP3 Am | TAG CGT TGG AGT GGA GAC | |
| 80 | BMP3 Bm | CCA ACC CCA CTT ACT ACG | 114 |
| 81 | ALX4 M1 | TTT TTC GGA GGC GAT AAG TTC | |
| 82 | ALX4 M2 | CGA ACC CGA CTC TTA ACG | 85 |
| 83 | ALX4 M beacon | (FAM)CGC GAT TGT CGG TCG TCG TTA AAG TAT CGC G(Dabcyl) | |
| 84 | ALX4 Am | CGT TTT CGT TCG TCG TTT GC | |
| 85 | ALX4 Bm | CGA ACC CGA CTC TTA ACG | 114 |
| 86 | SFRP2 M1 | GTT TTT CGG AGT TGC GCG C | |
| 87 | SFRP2 M2 | CCG AAA AAC TAA CAA CCG ACG | 98 |
| 88 | SFRP2 M beacon | (HEX)CGA CGT TTG TAG CGT TTC GTT CGC GTT GTT ACG TCG(Dabcyl) | |
| 89 | SFRP2 Am | GTT TTT CGG AGT TGC GC GC | |
| 90 | SFRP2 Bm | CTC TTC GCT AAA TAC GAC TCG | 124 |
| 91 | NEUROG1 M1 | GTT GAT TTG ATC GTC GGC | |
| 92 | NEUROG1 M2 | CTC GCC TAC AAA AAC CAC G | 64 |
| 93 | NEUROG1 M beacon | (HEX)CGC GAT GCC C+GA CC+G ATC TCC TAA ATC GCG(Dabcyl) | |
| 94 | NEUROG1 Am | GTT TAT ACG AGT TGA TTT GAT C | |
| 95 | NEUROG1 Bm | CTT AAC CTA ACC TCC TCG | 92 |
| 96 | NTPX2 M1 | AGG TTA GAG TGT CGA GTA GC | |
| 97 | NTPX2 M2 | TCG AAA ATC GCG TAC ACC G | 80 |
| 98 | NTPX2 M beacon | (HEX)CGC GAT CGG TG+C GGT TGT GAG A+CG GTG ATC GCG(Dabcyl) | |
| 99 | NTPX2 Am | TTC GGT AGG TTA GAG TGT C | |
| 100 | NTPX2 Bm | CTA TCG TCT CGA AAA TCG CG | 93 |
| 101 | TFPI2 M1 | TAT TTT TTA GGT TTC GTT TCG GC | |
| 102 | TFPI2 M2 | AAA CGA CCC GAA TAC CCG | 72 |
| 103 | TFPI2 M beacon | (HEX)CGC GAT CGT CGG T+CG GA+C GTT CGT TGA TCG CG(Dabcyl) | |
| 104 | TFPI2 Am | TAT TTT TTA GGT TTC GTT TCG GC | |
| 105 | TFPI2 Bm | CGA CTT TCT ACT CCA AAC G | 86 |
| 106 | BNC1 M1 | GTA GGT AGT TAG TTG GTT TTC | |
| 107 | BNC1 M2 | GAA ACA AAC GAC CCG AAA CG | 84 |
| 108 | BNC1 M beacon | (FAM)CGC GAT CGT ATT TA+C GGG AGT +CGG AGT TTG ATC GCG(Dabcyl) | |
| 109 | BNC1 Am | GTA GGT AGT TAG TTG GTT TTC | |
| 110 | BNC1 Bm | GCG AAA ATT CTC TAT ACG | 102 |
| 111 | CDKN2B M1 | TAT TGT ACG GGG TTT TAA GTC | |
| 112 | CDKN2B M2 | TTC CCT TCT TTC CCA CG | 109 |
| 113 | CDKN2B M beacon | (HEX)CGC GAT CGA +CGA +CGG GAG GGT AAT GGA TCG CG(Dabcyl) | |
| 114 | CDKN2B Am | GGT CGT TCG GTT ATT GTA C | |
| 115 | CDKN2B Bm | TTC CCT TCT TTC CCA CG | 119 |
| 116 | WNT5A M1 | CGT GGA ATA GTT GTT TGC | |
| 117 | WNT5A M2 | TTA AAA CAA AAC TAA AAT ACG | 135 |
| 118 | WNT5A M beacon | (HEX)CGC GAT CAA CCT AAT C+GA AAC +GCA ACT AAA GAT CGC G(Dabcyl) | |
| 119 | WNT5A Am | CGT GGA ATA GTT GTT TGC | |
| 120 | WNT5A Bm | CGA ACC TAA ACT CCC G | 153 |
| 121 | PENK M1 | AGG CGA TTT GAG TCG TTT TTA C | |
| 122 | PENK M2 | GAC AAC CTC AAC AAA AAA TCG | 113 |
| 123 | PENK M beacon | (HEX)CGC GAT CAA AGT TGT +CGG T+CG GGA GG ATC GCG(Dabcyl) | |
| 124 | PENK Am | CGC GTT ATT TCG GGA ATC | |
| 125 | PENK Bm | GAC AAC CTC AAC AAA AAA TCG | 134 |
| 126 | HIC1 M1 | TTC GGT TTT CGC GTT TTG TTC | |
| 127 | HIC1 M2 | CGA AAA CTA TCA ACC CTC G | 92 |
| 128 | HIC1 M beacon | (FAM)CGC GAC GGT CGT CGT TCG GGT TCG CG (Dabcyl) | |
| 129 | HIC1 Am | GAT ATA ACG TTT TTT TCG CGT C | |
| 130 | HIC1 Bm | ATA CCC GCC CTA ACG CCG | 142 |
| 131 | GSTP1 M1 | TCG GGG TGT AGC GGT C | |
| 132 | GSTP1 M2 | CCC AAT ACT AAA TCA CGA CG | 86 |
| 133 | GSTP1 M beacon | (HEX)CGCGAT GTC G+G+C GGG AGT TCG ATC GCG (Dabcyl) | |
| 134 | GSTP1 Am | AGG GCG TTT TTT TGC GGT C | |
| 135 | GSTP1 Bm | CCC AAT ACT AAA TCA CGA CG | 100 |
| 136 | *MESTv1 M1 | CGC GGT AAT TAG TAT ATT TC | |
| 137 | *MESTv1 M2 | GCT ACG ACA CTA CGC TTA CG | 67 |
| 138 | *MESTv1 M beacon | (HEX)CGC GAT CGG +TA+G T+TG +CGT TAT CGC G(Dabcyl) | |
| 139 | *MESTv1 U1 | TGT TGT GGT AAT TAG TAT ATT TT | |
| 140 | *MESTv1 U2 | CAA CCA CTC CAA CAT ACA CTA CA | 86 |
| 141 | *MESTv1 U beacon | (FAM)CGC GAG +TA+G T+TG +TG+T TT+T GTT CGC G(Dabcyl) | |
| 142 | **MESTv1 A | GGT TTT AAA AGT T/CGG TGT TTA TT | |
| 143 | **MEST1v1 B | CCI AAC AAC TAC AAC CAC TCC | 130 |
| | M1; methylation specific forward primer for the array (inner primer) | | |
| | M2; methylation specific reverse primer for the array (inner primer) | | |
| | M beacon; methylation specific probe | | |
| | Am; methylation specific forward primer for the nested/semi-nested PCR (outer primer/ 1. Round of PCR) | | |
| | Bm; methylation specific reverse primer for the nested/semi-nested PCR (outer primer/ 1. Round of PCR) | | |
| | * Hemimethylated reference gene MEST transcript variant 1 | | |
| | ** Un-methylated primer for the reference gene MEST transcript variant 1 | | |

### Examples

### Example 1

### Cell-free DNA Promoter Hypermethylation in Plasma as a Diagnostic Marker for Pancreatic Adenocarcinoma

### Methods

### Study design

This study was conducted as a cross sectional observational study of the cell-free DNA hypermethylation profile in plasma of patients with suspected or biopsy-verified pancreatic cancer admitted to the Department of Gastrointestinal Surgery, Aalborg University Hospital between February 2008 and February 2011. An additional benign control groups were patients with chronic pancreatitis treated at the hospital or at the outpatient clinic at Aalborg University Hospital from August 2013 until August 2014 and patients admitted with acute pancreatitis at the Department of Gastrointestinal Surgery, Aalborg University Hospital or the Department of General Surgery, Hospital of Vendsyssel from November 2013 until May 2015.

The study was approved by the Ethical Committee of Northern Jutland, Denmark (N-2013037) and registered in ClinicalTrails.gov: NCT02079363. All participants gave written informed consent.

### Participants

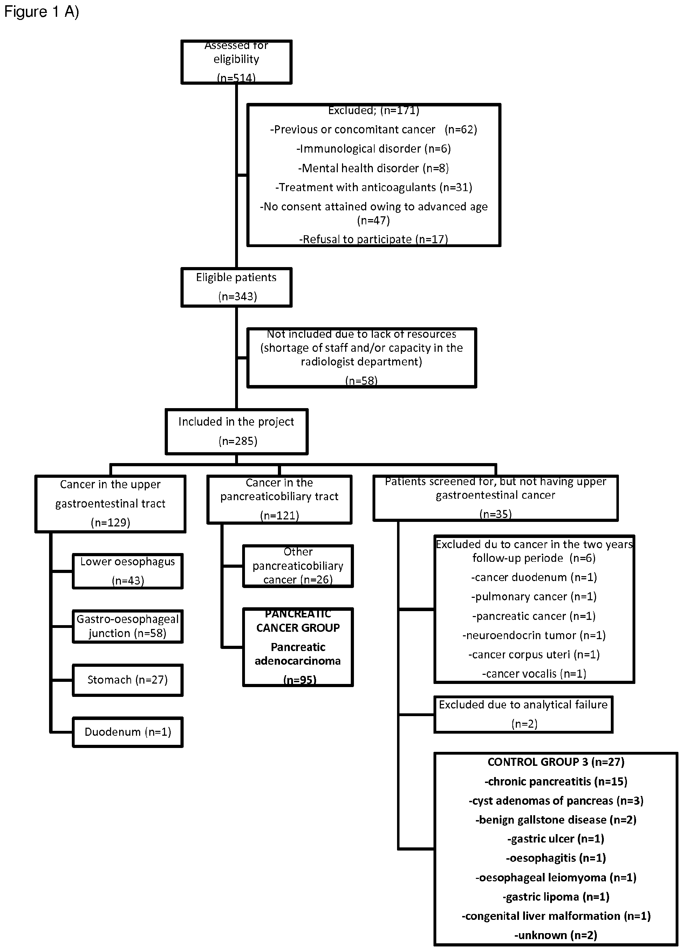

Patients with suspected or biopsy-verified upper gastrointestinal cancer were included prospectively and consecutively and had blood drawn on admission before diagnostic work-up and before any kind of treatment. Patients were divided into the following groups (figure 1a). Patients with pancreatic adenocarcinoma (cancer group) and patients screened for but not having upper gastrointestinal cancer (control group 3) were included in this study. Patients were excluded if they had previous (within three years) or concomitant cancer, known congenital thrombophilia, previous venous thromboembolism, connective tissue disease, or ongoing anticoagulant therapy. Patients with chronic pancreatitis (control group 1) had blood drawn during hospitalization or at a scheduled visit in the outpatient clinic. Patients diagnosed with acute pancreatitis (control group 2) were enrolled during the first three days of hospitalization. Previous cancer history was the only exclusion criterion for patients with acute or chronic pancreatitis.

### Blood sampling and analytical method

All blood samples were obtained by skilled technicians using venipuncture according to procedure recommended by the European Concerted Action on Thrombosis. Routine analyzes (C-reactive protein (CRP), leucocytes, alanine aminotransferase (ALT), alkaline phosphatase (ALP), amylase, bilirubin) were performed immediately afterwards. EDTA plasma for methylation analysis was centrifuged 20 min. (4000 rpm) at 4 °C and stored within two hours after sampling in a biobank at -80 °C until methylation analysis.

All methylation analyzes were performed by a single skilled laboratory scientist. *Extraction and deamination:* Plasma nucleic acids were extracted using the EasyMag platform (Biomerieux) according to manufacturer's instruction. Four-hundred-fifty- 1000 µl EDTA plasma was used for the extraction and purified nucleic acids were eluted in 35 µl elution buffer (Biomerieux). Five µl was used for quantitation of extracted DNA, the rest was deaminated as described previously by our group. In brief, 30 µl DNA extract was mixed with 60 µl deamination solution, deaminated for 10 min at 90 °C, followed by purification using EasyMag and eluted in 25 µl 10 mM KOH.

*PCR:* In order to expand the amount of relevant deaminated DNA a first round PCR was conducted using a mix of outer primers (Table 2) for all promoter regions investigated. Subsequently, a second round of PCR was carried out using each of the inner primers and probes (Table 2) in individual reactions.

**Table 3. Variables included in the study. All variables are analyzed by simple logistic regression comparing the pancreatic cancer group and control groups 1+3**

| Gene | p-value | AUC |
|---|---|---|
| **ALX4** | **0-0034** | **0·57** |
| **APC** | **9·67 × 10⁻⁶** | **0·65** |
| **BMP3** | **2·64 × 10⁻⁶** | **0·64** |
| **BNC1** | **5·02 × 10⁻⁷** | **0·65** |
| BRCA1 | 0·6804 | 0·51 |
| CDKN2A | 0·1652 | 0·52 |
| CDKN2B | 0·0757 | 0·53 |
| CHFR | 0·4668 | 0·51 |
| **ESR1** | **0·0095** | **0·58** |
| EYA2 | 0·0778 | 0·54 |
| GSTP1 | 0·2323 | 0·51 |
| **HIC1** | **0·0097** | **0·55** |
| **MESTv2** | **0·0004** | **0·62** |
| MGMT | 0**·**2778 | 0·51 |
| MLH1 | 0**·**3448 | 0·52 |
| **NPTX2** | **4·34 × 10⁻⁵** | **0·64** |
| NEUROG1 | 0**·**3969 | 0**·**52 |
| **RARB** | **0·0348** | **0·57** |
| **RASSF1A** | 1·4 **× 10⁻⁶** | **0·65** |
| **SFRP1** | **0·0001** | **0·62** |
| **SFRP2** | **0·0197** | **0·57** |
| **SEPT9v2** | **0·0029** | **0·56** |
| **SST** | **8·69 × 10⁻⁵** | **0·64** |
| **TFPI2** | **7·96 × 10⁻⁵** | **0·60** |
| **TAC1** | **3·63 × 10⁻⁵** | **0·64** |
| **VIM** | ^{∗} | 0**·**5 |
| **WNT5a** | **0·0234** | **0·54** |
| **PENK** | * | 0·5 |
| sex | 0·5750 | 0·52 |
| age60 | **4·58 × 10⁻⁶** | 0·66 |
| **age65** | **1·14 × 10⁻⁶** | **0·67** |
| age70 | 6·06 × 10⁻⁵ | 0·62 |

| | | |
|---|---|---|
| Bold marks the genes, where there is significant difference (p<0**·**05) in hypermethylation frequency between the cancer group and control groups 1+3. Control group 1; patients with chronic pancreatitis. Control group 3; patients screened for but not having pancreatic cancer. AUC; area under the receiver operating characteristic curve. ^{∗}VIM and PENK could not be evaluated by logistic regression because none of the patients in the control group had hypermethylation of the two genes, however chi-square test found significant difference between the cancer group and the control group 1+3. Despite that, VIM and PENK were excluded from the following analysis because only few cancer patients had VIM or PENK hypermethylation. | | |

*First round PCR amplification:* In order to expand the amount of relevant deaminated DNA a first round PCR was conducted using a mix of outer primers (table 2) for all promoter regions investigated. The reaction buffer for each sample consisted of 25 µl containing; PCR stock, 13 µM MgCI2, 0**·**6 mM dNTP, 250 nM of each outer primer (table 2) 1**·**5 U Taq polymerase (Bioline), and 0**·**3 U UNG (Invitrogen). The first round reaction mix was distributed to individual 200 µl PCR tubes and incubated for 5 min at 37 °C (UNG activity), followed by 95 °C for 5 min. and cooled to room temperature. Twenty-five ul purified deamination product was added to each tube containing first round reaction mix. PCR was performed for 20 rounds: 92 °C for 15 sec., 55 °C for 30 sec., and 72 °C for30 sec.

*Second round PCR:* Ten µl mix containing 0.4 µM inner primers and probes (table 2) were distributed in 30 individual wells in a 96 well PCR plate. Ten µl first round PCR product were added to 710 µl reaction mix containing; PCR stock, 250 µM dNTP, 10 µM MgCI2, and 15 U Taq polymerase (Bioline). Twenty µl of the reaction mix were added to each of the 30 wells containing primers and probes. Real time PCR was carried out for 45 rounds of 94 °C for 15 sec., 55 °C for 30 sec. (anneal and detection), and 72 °C for 30 sec.

*Gene panel:* Twenty-eight genes (Table 3), which all have the potential to participate in development of pancreatic cancer, were selected for methylation analysis. *Primer and probe design:* "Beacon Designer" was used to design potential primers and probes for the selected genes. Primers were designed to be rich on CpG's and to be located up-stream of exon one which was interpreted as the promoter regions of the genes. The aim was to design PCR products with a length less than 140-150 base pairs, since the cell-free DNA fragments most likely have a length of 160 base pairs consistent with nucleosomal DNA size (table 2).

### Outcome

The primary outcome of the prediction model was pancreatic adenocarcinoma.

### Statistical methods

Each gene in the gene panel was analyzed as a binary variable.

*Validation of dichotomous data:* We calculated differences between the threshold cycle (Ct) values of the hemimethylated reference gene MEST transcript variant 1 and Ct values of each gene for which Ct > 0. To assess the amount of information lost in the dichotomization, histograms of the differences for the cancer group and control group 1 combined with control group 3 were produced. No clear indication of difference in the two distributions was observed. This was interpreted as an indication that no significant amount of information was lost by dichotomizing the genes as hypermethylated or nonmethylated disregarding the observed Ct value.

The methylation frequency of each gene and the (exact) 95% confidence interval (CI) were calculated for each group. The mean number of hypermethylated genes in each group and the 95% CI was calculated. The means were compared as numerical data with nonparametric Wilcoxon rank sum test. P-values less than 0**·**05 were considered statistically significant.

### Prediction model development

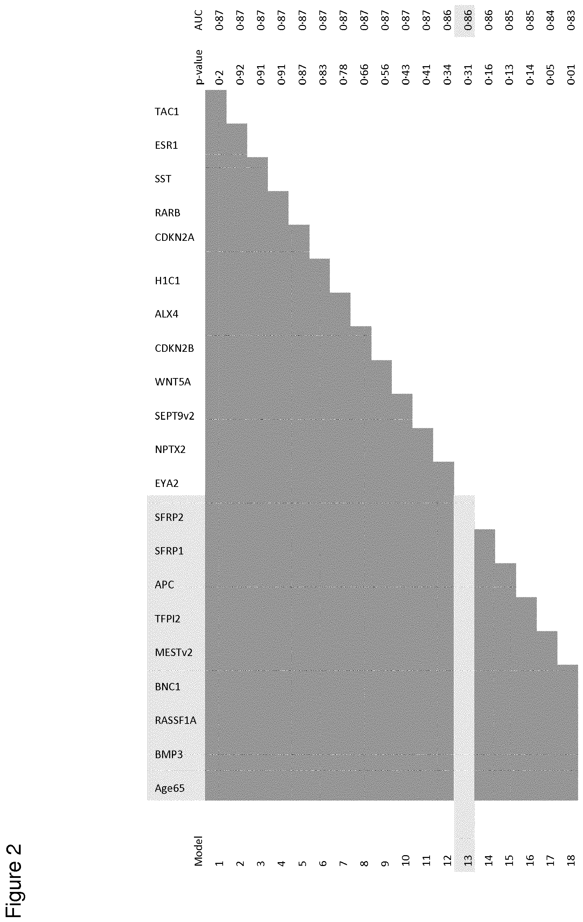

*1. Screening of each individual variable* as a *diagnostic marker for pancreatic cancer:* Logistic regression was performed separately for each gene in the gene-panel and for smoking status, gender and age>65. The p-value and the area under the receiver operating characteristic curve (AUC) were calculated (Table 3).
2. *The selection of variables:* Variables having a p-value less than 0**·**2 were selected for further analysis.
3. *Model selection:* Stepwise backward elimination in logistic regression models was performed to select the relevant variables using 0**·**05 as the significance level for removal from the model. For each intermediate model, the AUC value was calculated (figure 2).
*4. Determination of the best model:* The decision was based on model complexity combined with model performance according to the AUC.
5. *Interactions between the variables:* The significance of interactions between all pairs of variables were assessed in the final model. Interactions with a p-value less than 0**·**01 were considered statistically significant.
6. *Validation:* To account for optimism in the internal validation of discriminative model performance (measured by the AUC) *"leave pair out cross validation"* was used. For calibration performance, *"Hosmer-Lemeshow"* test was performed.
*7. Probability score:* For each patient a probability score was calculated.

All data were analyzed using STATA 14**·**0 software.

### Results

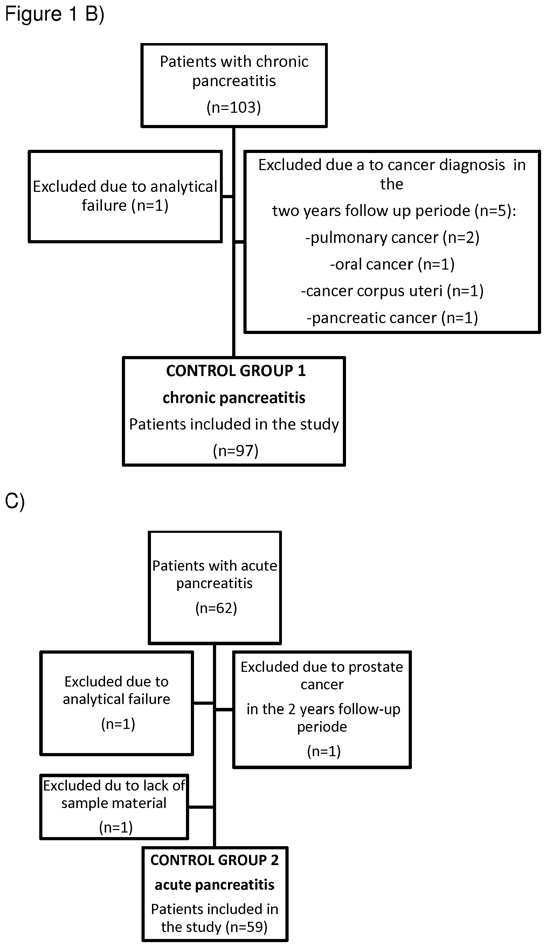

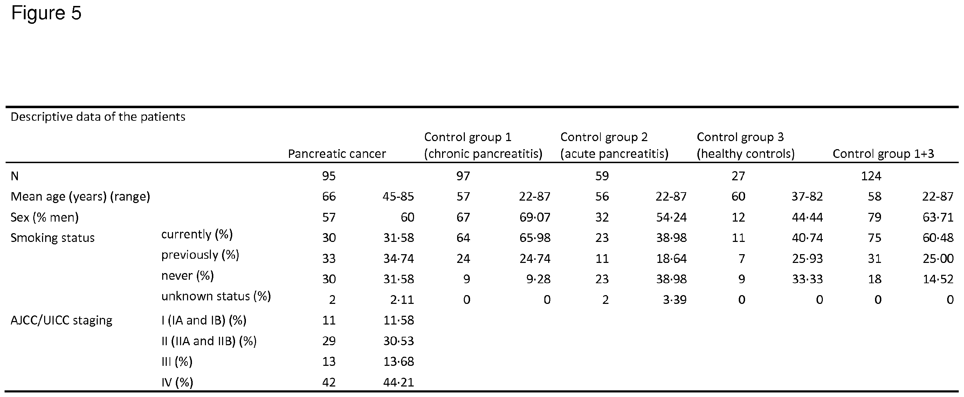

Ninety-five patients with pancreatic adenocarcinoma were included in the study (figure 1a). After diagnostic work-out (gastroscopy, endoscopic ultrasound, magnetic resonance (MR) or CT scan) and no evidence of malignancy found, 35 patients were categorized as patients screened for but not having pancreatic adenocarcinoma (control group 3). Eight patients were subsequently excluded from this group, see figure 1a. Two additional control groups of patients with benign pancreatic disease were included. In total, 103 patients with chronic pancreatitis (control group 1) (figure 1b) and 62 patients with acute pancreatitis (control group 2) (figure 1c) were included. Subsequently, six patients from control group 1 (figure 1b) and three patients from control group 2 (figure 1c) were excluded. Descriptive data of the four groups can be seen in Figure 5.

The methylation frequency of each gene is presented in table 1. The mean number of methylated genes of the whole gene panel (28 genes) was 8**·**41 (95% CI 7**·**62-9**·**20) for the cancer group compared to 4**·**34 (95% CI 3**·**85-4**·**83) for patients with chronic pancreatitis (control group 1), 4**·**89 (95% CI 4**·**07-5**·**71) for patients screened for but not having pancreatic cancer (control group 3) and 5**·**34 (95% CI 4**·**76-5**·**91) for patients with acute pancreatitis (control group 2). The difference was highly statistically significant between the cancer group and the three benign control groups (Table 4).

**Table 4. Mean number of hypermethylated genes in each group.**

| Group | N | Mean number of methylated genes | 95% CI | P-value |
|---|---|---|---|---|
| Pancreatic cancer | 95 | 8**·**41 | (7·62 - 9·20) | |
| Control group 1; chronic pancreatitis | 97 | 4**·**34 | (3**·**85- 4**·**83) | |
| Control group 2; acute pancreatitis | 59 | 5**·**34 | (4·77- 5·91) | |
| Control group 3; healthy controls | 27 | 4·89 | (4**·**07- 5**·**71) | |
| Control group 1+3 | 124 | 4-46 | (4·04- 4·88) | <0.0001^{∗} |
| Control group 1+2+3 | 183 | 4·74 | (4·40- 5**·**08) | <0.0001^{∗∗} |
| The means were compared as numerical data with nonparametric Wilcoxon rank sum test. P-values less than 0**·**05 were considered statistically significant. | | | | |
| ^{∗} Significant difference between patients with pancreatic cancer and control group 1+3 | | | | |
| ^{∗∗} Significant difference between patients with pancreatic cancer and control group 1+2+3 | | | | |

*Model development:* In the following analyzes we chose to combine control group 1 and 3. The combined group has symptoms resembling those of pancreatic cancer, which makes a biomarker to distinguish these from pancreatic cancer of utmost relevance. For the rest of the analysis patients with acute pancreatitis were left out, since a clinical picture of acute inflammation is rarely seen in pancreatic cancer. There was a highly significant difference (p < 0**·**001) between the cancer group and control group 1+3 in hypermethylation frequency of ten genes (APC, BMP3, BNC1, MESTv2, NPTX2, RASSF1A, SFRP1, SST, TFPI2, and TAC1) (Table 3) and significant difference (p < 0**·**05) in seven genes (ALX4, ESR1, HIC1, RARB, SFRP2, SEPT9v2, and WNT5A) (Table 1). VIM and PENK could not be evaluated by logistic regression as none of the patients in the control group had hypermethylation of these two genes (Table 3 and table 4). There was no significant difference in gender, why this variable was excluded from the following analysis. Smoking however, was a preventive factor for cancer when comparing patients with pancreatic cancer and patients with chronic pancreatitis. Smoking was therefore excluded from the model, since it is a known risk factor for cancer. By dividing the patients into groups according to age; >65 year, <=65 year, a statistically significant difference was found. Consequently, age>65 was included in the multivariable logistic regression analysis.

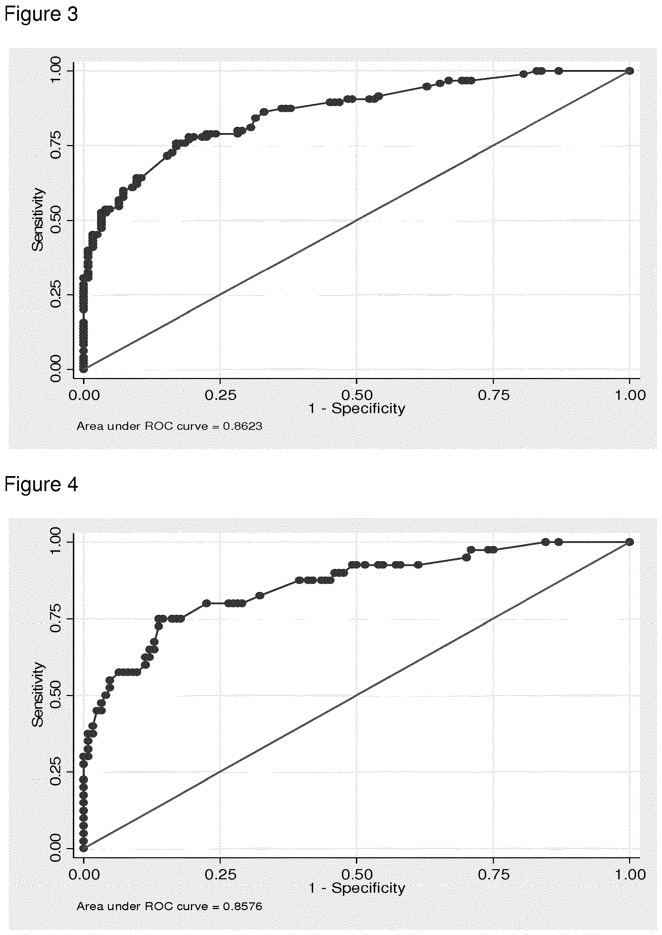

All genes with an individual p-value below 0**·**20 (20 genes out of 28 examined genes) and age>65, were included in multivariable logistic regression model. Backward stepwise selection was performed (figure 2). The initial model (model 1) with 20 genes had an AUC of 0**·**87 (figure 2). Removing the 12 least significant genes form the model, leaving eight genes (model 13; age>65, BMP3, RASSF1A, BNC1, MESTv2, TFPI2, APC, SFRP1, and SFRP2) resulted in an AUC of 0·86 (95% CI 0**·**81-0**·**91) (figure 2 and 3). The mean probability for having pancreatic cancer was of 0**·**67 (0**·**61-0**·**72) for cancer patients and 0**·**26 (0**·**22-0**·**29) for control groups 1+3. Model 13 was determined as the model with best performance (probability cut point of 0·50; sensitivity 75**·**79% and specificity 83·06%). There were no statistically significant interactions between variables in model 13. The model was well calibrated (p=0**·**40) and had an estimated optimism in AUC of 0**·**03.

Forty patients had stage I or II tumors. Model 13 had an apparent AUC of 0·86 (95% CI 0**·**79-0**·**92) for Stage I/II tumors (probability cut point of 0·50; sensitivity 72**·**50% and specificity 83**·**06%) (figure 4) with an optimism in AUC of 0**·**06.

### Discussion

We examined cell-free DNA promoter hypermethylation of 28 genes in plasma of patients with pancreatic cancer (n=95) and compared it to three different control groups. The gene-panel was composed of genes previously tested in relation to pancreatic cancer (BNC1, NPTX2, PENK, CDKN2A, RASSF1A, SFRP1 (SARP2), APC, BRCA1, CDKN2B, ESR1, MGMT, MLH1, RARB) and genes which have not earlier been examined in plasma from patients with pancreatic cancer (ALX4, BMP3, CHFR, EYA2, GSTP1, HIC1, SFRP2, MESTv2, NEUROG1, SEPT9v2, SST, TFPI2, TAC1, VIM, WNT5a). This is the first study to examine cell-free DNA hypermethylation in a wide selection of genes by methylation specific PCR in a large group of patients with either benign or malignant pancreatic disease.

Statistically significant difference in the hypermethylation status in 19 out of the 28 genes was found when comparing pancreatic cancer patients and a control group containing patients screened for, but not having pancreatic cancer as well as in patients with chronic pancreatitis. Cell-free DNA hypermethylation of BMP3, MESTv2, SST, TFPI2, TAC1, ALX4, HIC1, SFRP2, SEPT9v2 and WNT5A is not previously described in the literature in relation to pancreatic cancer. Yi et al. described BNC1 hypermethylation to have a sensitivity of 79% and a specificity of 89% when comparing pancreatic cancer and healthy individuals. Park et al. examined hypermethylation of a small gene-panel (NPTX2, RASSF1A, SFRP1, UCHL1, PENK and p16 (CDKN2A)) by methylation specific PCR. The gene-panel could differentiate pancreatic cancer from healthy controls, however it was not able to discriminate benign and malignant pancreatic disease.

The example shows that patients with pancreatic cancer have a higher level of hypermethylated genes in plasma derived cell-free DNA compared to relevant control groups. In consistence with previous studies, our gene panel did not demonstrate a single gene which had the potential of being used as a diagnostic marker for pancreatic cancer. This suggests that a larger gene panel is needed to achieve sufficient accuracy. We developed a prediction model (age>65, BMP3, RASSF1A, BNC1, MESTv2, TFPI2, APC, SFRP1, and SFRP2) which was able to differentiate between pancreatic cancer and a large relevant control group consisting of patients with chronic pancreatitis or patients referred to the hospital with symptoms of pancreatic cancer. The AUC was high and the predictive value of our model is superior to the predictive value of CA-19-9, in particular keeping in mind that CA-19-9 is highly dependent on the Lewis blood group status of the patients. Only Le^{a+b-} or Le^{a-b+} individuals are able to express CA-19-9, and not Le^{a-b-} individuals which represent 5-10 % of the caucasian population. CA-19-9 could in a recent study differentiate patients with stage I-II pancreatic cancer from patients with chronic pancreatitis with an AUC of 0**·**77 (sensitivity of 53% and a specificity of 91**·**9%) and pancreatic cancer patients from patients with benign biliary obstruction with an AUC of only 0**·**65.⁶ Our study included patients with stage I-IV pancreatic cancer. It is most important to diagnose patients with stage I and II disease as early detection at this stage of disease have the potential to improve outcome of surgery. We tested our model on stage I and II disease and found an AUC of 0**·**86. This shows that the performance of the prediction model is independent of cancer stage. Alteration in DNA hypermethylation is detectable in plasma even in the early stage disease and thereby potential usable as early diagnostic marker.

In order to further distinguish DNA hypermethylation related to malign and benign pancreatic disease, patients with acute pancreatitis were included. The aim was achieve more basic knowledge about hypermethylated DNA during the cause of an acute pancreatic inflammatory reaction, which not earlier have been described in literature. Our study shows that DNA hypermethylation takes place during acute pancreatitis, however the changes are more pronounced in patient with pancreatic cancer.

The data provided herein has several strengths. We tested cell-free DNA hypermethylation of a broad gene panel in plasma from a large group of patients with pancreatic cancer all included prospectively and consecutively, before the diagnosis and before any treatment. We include a large relevant control group of patients with either benign pancreatic disease or symptoms mimicking pancreatic cancer. We used an optimized method of methylations specific PCR, with an improved sensitivity compared to previous methods. However, the study also has some limitations. In the end of the analyzes we discovered that the use of UNG (Invitrogen) had a tendency to lower the sensitivity compared to the use of COD UNG (ArcticZymes). All our samples are analyzed using UNG (Invitrogen), since it was not possible to repeat all analyzes with COD UNG (ArcticZymes) due to lack of sample material. Furthermore, patients are not matched according to age, which one should be aware of because epigenetic changes can be a part of aging. To address this problem we incorporated age in our prediction model.

### Conclusion

This example shows statistically significant differences in hypermethylation of several genes between malignant and benign pancreatic disease. Pancreatic cancer patients have a higher number of hypermethylated genes compared to patients with benign pancreatic disease. Based on the data provided in this example, alterations in cell-free DNA hypermethylation can be applied as blood based biomarkers for pancreatic cancer.

### Example 2

### Cell-free DNA Promoter Hypermethylation in Plasma as a Prognostic Marker for Pancreatic Adenocarcinoma

### Methods

### Study design

This study was conducted as a cross sectional observational study of plasma derived cell-free DNA hypermethylation from patients with pancreatic cancer, who were admitted to the Department of Gastrointestinal Surgery, Aalborg University Hospital from February 2008 until February 2011.(21) All participants gave written informed consent. The study was registered in ClinicalTrails.gov: NCT02079363 and approved by the Research Ethics Committee for the North Denmark Region (N-2013037).

### Participants

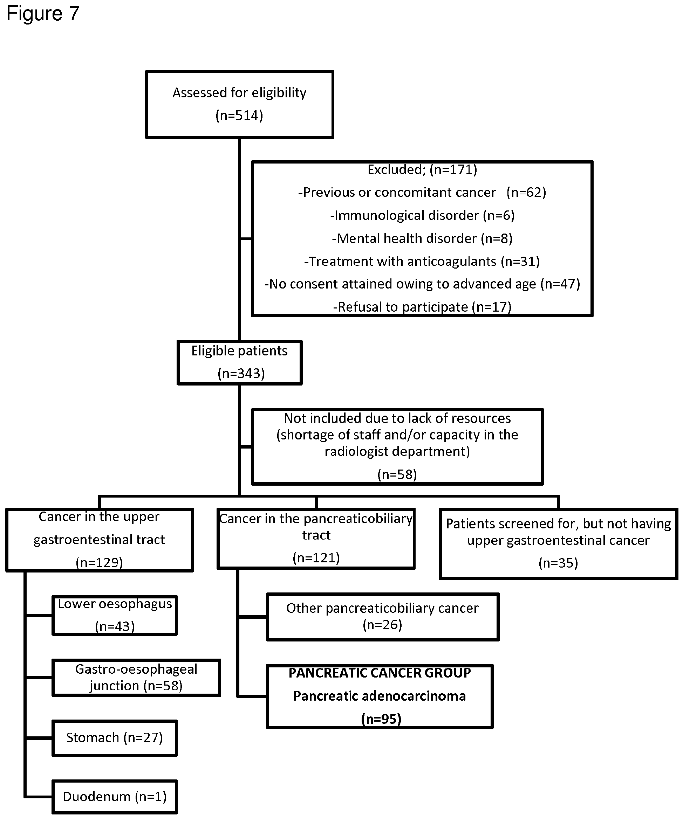

Patients with suspected or biopsy-verified upper gastrointestinal cancer were included prospectively and consecutively. Blood samples were drawn on admission before the diagnostic work-up and before any kind of treatment. Only patients with pancreatic adenocarcinoma were included in this study (figure 7). Patients were excluded if they had previous (within three years) or concomitant cancer, known congenital thrombophilia, previous venous thromboembolism, connective tissue disease, or ongoing anticoagulant therapy. Data from the same patients were published in a previous study concerning DNA hypermethylation as a diagnostic marker for pancreatic cancer

### Diagnosis and stage classification.

CT or PET scan of thorax and abdomen were performed in the diagnostic work up of all patients. Histopathological analysis of biopsy specimens obtained by either endoscopic or laparoscopic ultrasound confirmed the cancer diagnosis. Patients were staged according to AJCC TNM classification 7^{th}. T and N categories were determined by histopathological analysis for patients who underwent intended curative surgery. If surgery was not performed, the final clinical decision determined the T and N categories. Cancer stage and treatment of all patients were discussed at a multidisciplinary team conference, where a final decision was made.

### Blood sampling and analytical method

Blood samples were obtained by skilled technicians. EDTA plasma for methylation analysis was centrifuged 20 min. (4000 rpm) at 4°C and stored within two hours after sampling in a biobank at -80°C until further analysis.

All methylation analyzes were performed by a single skilled laboratory scientist. Extraction and deamination of cell-free DNA was performed as described by our group. A first round PCR amplification was conducted in order to expand the amount of relevant deaminated DNA. A mix of outer primers for all the investigated promoter regions was used (table 2). Afterwards, a second round of PCR was carried out using each of the inner primers and probes in individual reactions (table 2).

The gene panel consisted of 28 genes (table 1), all having the potential to participate in the development of pancreatic cancer.

### Outcome

The primary outcome of the prognostic prediction models were cancer stage according to AJCC staging. Models to differentiate (stage I, II, III vs IV), (stage I, II vs III, IV), (stage I, IIa vs IIb) and (stage I, II vs III) was developed.

### Statistical analysis methods

Each gene in the gene panel was analyzed as binary variables. Validation of dichotomous data was described in our previous study.

Patients were divided into groups according to AJCC staging. The mean of number of hypermethylated genes and the (exact) 95% confidence interval (CI) was calculated for each group. The means were compared as numerical data with nonparametric Wilcoxon rank sum test. A p-value less than 0.05 was considered statistically significant.

### Prognostic prediction model development:

*1. Screening of each individual variable* as a *prognostic marker for pancreatic cancer staging:* Logistic regression was performed separately for each gene in the gene panel and for age>65, gender and ASA-score. The p-value and the area under the receiver operating characteristic curve (AUROC) were calculated (Supplementary Table 3).
*2. The selection of variables:* Variables having a p-value less than 0.3 were selected for further analysis.
3. *Model selection:* To select the relevant variables stepwise backward elimination in logistic regression models was performed using 0.10 as the significance level for removal from the model. For each intermediate model, the AUROC value was calculated.
*4. Determination of the best model:* Model performance according to the AUROC combined with model complexity determined the best model.
5. *Interactions between the variables:* The significance of interactions between all pairs of variables were assessed in the final model. Interactions with a p-value less than 0.01 were considered statistically significant.
6. *Validation: "Leave pair out cross validation"* was used(23) to account for optimism in the internal validation of discriminative model performance (measured by the AUROC). *"Hosmer-Lemeshow"test* was performed for calibration performance.
*7. Probability score:* For each patient a probability score was calculated.

All data were analyzed using STATA 14.0 software.

### Results

Ninety-five patients with pancreatic adenocarcinoma were included in the study (figure 1). Descriptive data of the patients is shown in Table 5.

**Table 5. Demographic data of patients with pancreatic adenocarcinoma (n=95)**

| *AJCC stage^{∗}* | *I(Iα+Ib)* | *II (IIa+IIb)* | *III* | *IV* |
|---|---|---|---|---|
| N | 11 | 29 | 13 | 42 |
| Age (mean) (sd) | 70 (10,81) | 67 (8,21) | 65 (8,25) | 65 (9,21) |
| Sex (men:women) | 6:5 | 19:10 | 10:3 | 22:20 |
| ASA 1 | 4 36% | 14 48% | 8 62% | 0 0% |
| ASA 2 | 4 36% | 11 38% | 3 23% | 18 43% |
| ASA 3 | 3 27% | 4 14% | 2 15% | 12 29% |
| * American Joint Committee on Cancer (AJCC) stage classification according T (primary tumor), N (regional lymph nodes) and M (distant metastasis). | | | | |

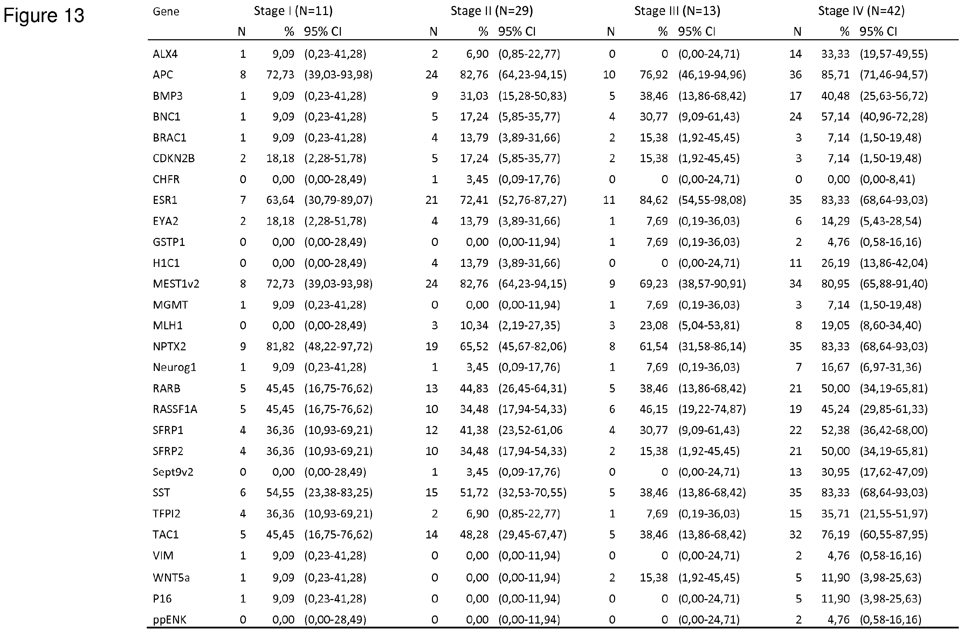

The methylation frequencies of each gene in each cancer stage are listed in figure 13. The mean number of methylated genes of the whole gene panel (28 genes) was 7.09 (5.51-8.66)) for patients with stage I (IA and IB) disease compared to 7.00 (5.93-8.07) for patients with stage II (IIA and IIB) disease, 6.77 (5.08-8.46) for patients with stage III disease and 10.24 (8.88-11.60) for patients with stage IV disease. There was no significant difference in the mean number of methylated genes in stage I, II and III disease. The difference was highly statistically significant between stage (I, II and III) and stage (IV) disease (Table 6).

**Table 6. Mean number of methylated genes according to AJCC stage***

| *AJCC stage^{∗}* | *N* | *Mean* | *95% CI* | | *P* |
|---|---|---|---|---|---|
| I (IA and IB) | 11 | 7.09 | | (5.52 8.67) | |
| II (IIA and IIB) | 29 | 7.00 | | (5.93 8.07) | |
| III | 13 | 6.77 | | (5.08 8.46) | |
| IV | 42 | 10.24 | | (8.88 11.60) | |
| I and II | 40 | 7.03 | | (6.17 7.88) | |
| III and IV | 55 | 9.42 | | (8.26 10.58) | 0,0078^{∗∗} |
| I and II and III | 53 | 6.96 | | (6.23 7.70) | 0,0002^{∗∗∗} |
| ** American Joint Committee on Cancer (AJCC) stage classification according T (primary tumor), N (regional lymph nodes) and M (distant metastasis). | | | | | |
| CI; confidence interval | | | | | |
| **Significant difference between stages I, II vs III,IV | | | | | |
| ***Significant difference between stages I, II, III vs IV | | | | | |

### Stage I, II, III vs IV

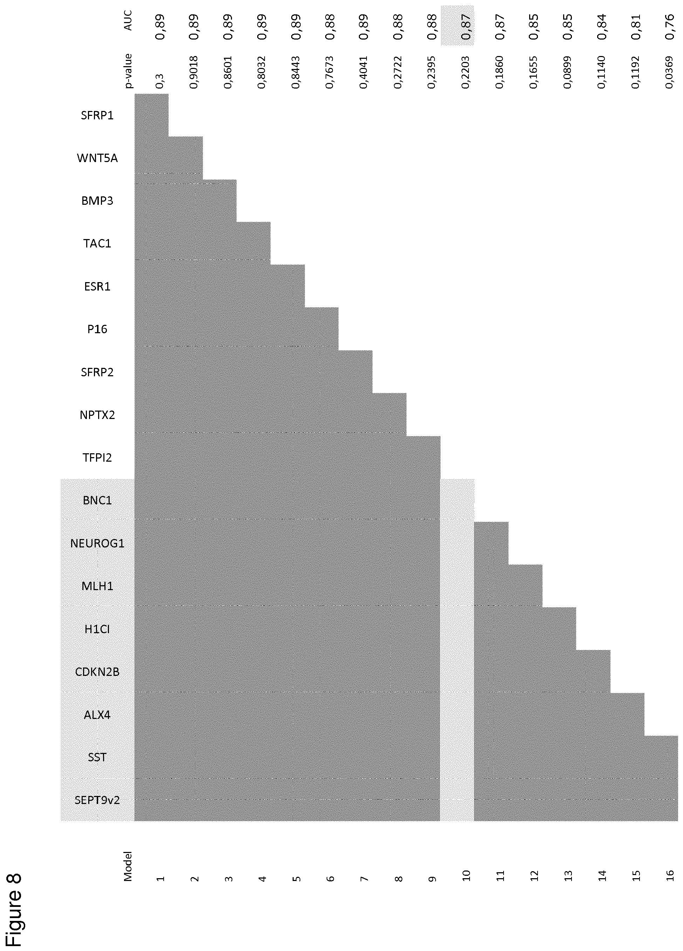

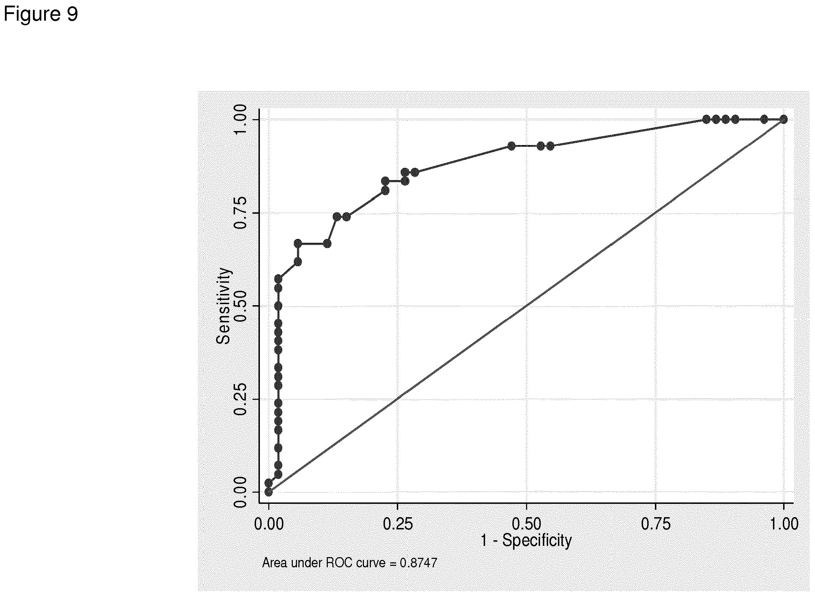

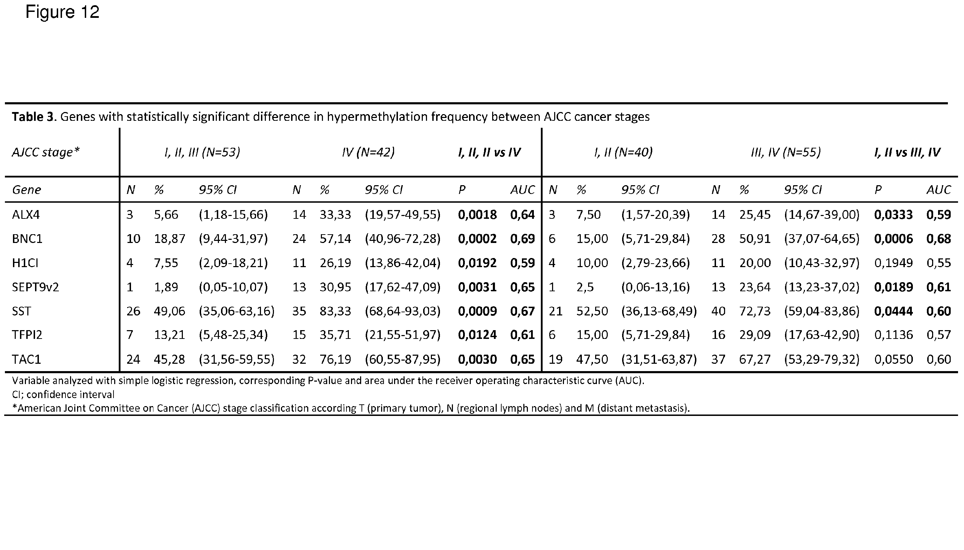

In the following analyzes patients with stage I, II or III (n = 53) disease are pooled and compared to stage IV (n=42) disease to develop a model to differentiate between patients having pancreatic cancer with distant metastasis and pancreatic cancer patients without distant metastasis. There was significant difference between stage I, II and III and stage IV in hypermethylation frequency of seven genes (ALX4, BNC1, HIC1, Sept9v2, SST, TFPI2 and TAC1) (figure 12 and table 7). There was no statistical significant difference in gender, age or ASA-score between the groups. There was a tendency towards more patients with distant metastasis having an ASA-score of three, despite that ASA-score, age and gender were excluded from the further analysis. All genes with an individual p-value below 0.30 (17 genes out of 28 examined genes) (tTable 7), were included in the multivariable logistic regression model. Backward stepwise selection was performed. The stepwise selection of genes is clarified with the corresponding AUROC (figure 8). The initial model (model 1) with the 17 genes had an AUROC of 0.8886 (figure 8). Removing the nine least significant genes form the model, leaving eight genes in the panel (Model 10; SEPT9v2, SST, ALX4, CDKN2B, HIC1, MLH1, NEUROG1, BNC1) resulted in an AUROC of 0.87 (figure 2 and 3). Mean probability of 0.26 (0.20-0.31) for cancer patients with stage I, II or III compared to the mean probability of 0.67 (0.59-0.76) for patients with stage IV disease. Model 10 was determined as the model with best performance (probability cut point of 0.55; sensitivity 73.81% and specificity 86.79%) (figure 9). There was no statistical significant interactions in Model 10. The model was well calibrated (p=0.18) and had an estimated optimism in AUROC of 0.05.

**Table 7. Variables included in the study. All variables are analyzed by simple logistic regression.**

| *AJCC stage* | *I, II, II vs IV* | | *I, II vs III, IV* | |
|---|---|---|---|---|
| *Gene* | *P* | *AUC* | *P* | *AUC* |
| ALX4 | **0,0018** | **0,64** | **0,0333** | **0,59** |
| APC | 0,4163 | 0,53 | 0,6485 | 0,52 |
| BMP3 | 0,2143 | 0,56 | 0,1296 | 0,58 |
| BNC1 | **0,0002** | **0,69** | **0,0006** | **0,68** |
| BRAC1 | 0,3458 | 0,53 | 0,5943 | 0,52 |
| CDKN2B | 0,1636 | 0,55 | 0,2304 | 0,54 |
| CHFR | * | 0,50 | * | 0,50 |
| ESR1 | 0,2590 | 0,55 | 0,1182 | 0,57 |
| EYA2 | 0,8793 | 0,51 | 0,7505 | 0,51 |
| GSTP1 | 0,4419 | 0,51 | * | 0,50 |
| HIC1 | **0,0192** | 0,59 | 0,1949 | 0,55 |
| MESTv2 | 0,6699 | 0,52 | 0,8301 | 0,51 |
| MGMT | 0,4723 | 0,52 | 0,3259 | 0,52 |
| MLH1 | 0,2960 | 0,54 | 0,1019 | 0,56 |
| NPTX2 | 0,0908 | 0,58 | 0,3665 | 0,54 |
| NEUROG1 | 0,0965 | 0,56 | 0,1524 | 0,55 |
| RARB | 0,5218 | 0,53 | 0,8264 | 0,51 |
| RASSF1A | 0,5822 | 0,53 | 0,4388 | 0,54 |
| SFRP1 | 0,1551 | 0,57 | 0,4814 | 0,54 |
| SFRP2 | 0,0511 | 0,60 | 0,5015 | 0,53 |
| SEPT9v2 | **0,0031** | 0,65 | **0,0189** | 0,61 |
| SST | **0,0009** | 0,67 | **0,0444** | 0,60 |
| TFPI2 | **0,0124** | 0,61 | 0,1136 | 0,57 |
| TAC1 | **0,0030** | 0,65 | 0,0550 | 0,60 |
| VIM | 0,4419 | 0,51 | 0,7559 | 0,51 |
| WNT5a | 0,2865 | 0,53 | 0,1110 | 0,55 |
| CDKN2A | **0,0807** | 0,55 | 0,2227 | 0,53 |
| PENK | * | 0,50 | * | 0,50 |
| sex | 0,1789 | 0,57 | 0,6716 | 0,52 |
| age65 | 0,3544 | 0,55 | 0,2050 | 0,56 |
| Bold marks the genes, where there is significant difference (p<0·05) in hypermethylation frequency between the two | | | | |
| AUC; area under the receiver operating characteristic curve. | | | | |
| ^{∗}Genes which could not be evaluated by logistic regression because one of the groups did not contain any patients with hypermethylation of this specific gene. | | | | |

### Stage I, II vs III, IV

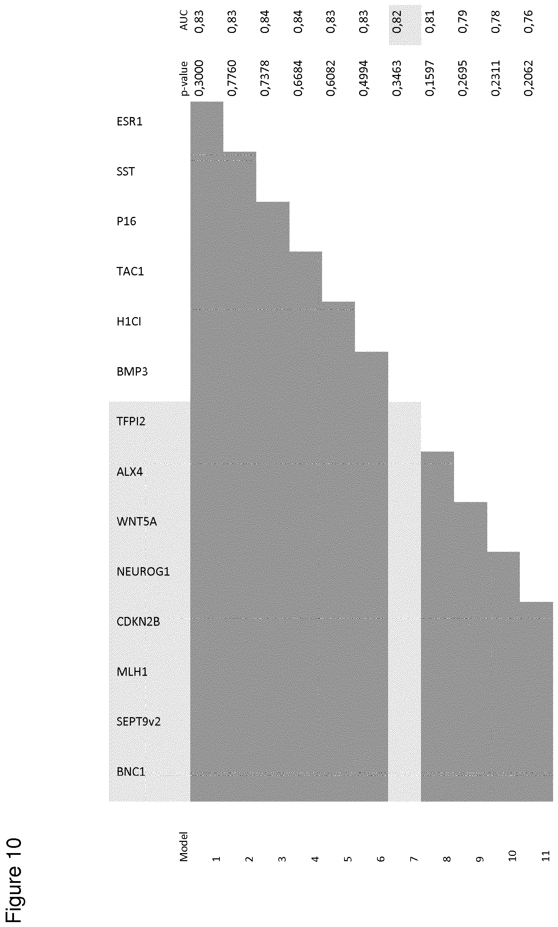

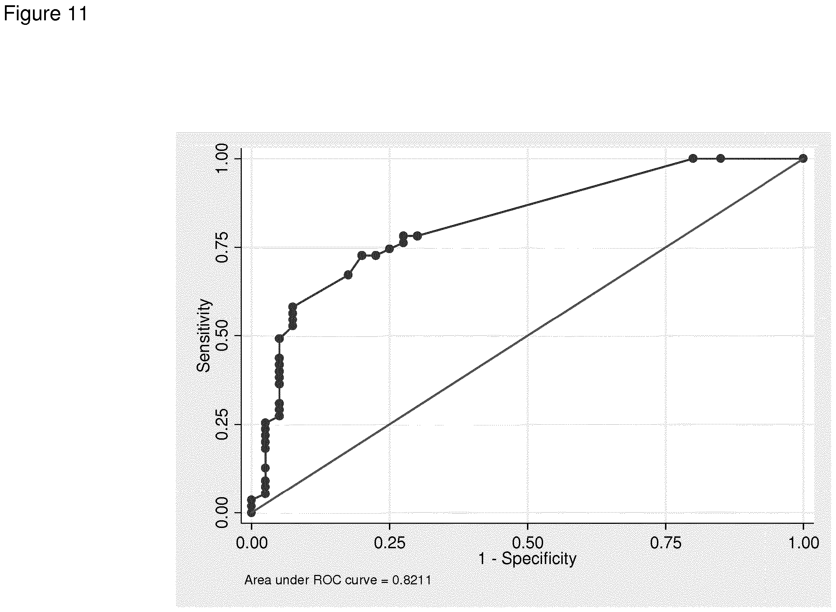

Patients with stage I or II (n = 40) disease are combined and compared to stage III or IV (n=55) disease to develop a model to differentiate between patients with pancreatic cancer which are potentially resectable (stage I or II) and patients with unresectable pancreatic cancer (stage III or IV). There was statistical significant difference (p < 0.05) between stage I or II and stage III or IV in hypermethylation frequency of four genes (ALX4, BNC1, Sept9v2, SST) (figure 12 and table 7) and no statistical significant difference in gender, age or ASA-score. All genes with an individual p-value below 0.30 (14 genes out of 28 examined genes) (table 7), were included in the multivariable logistic regression analysis using backward stepwise selection. Model 1 with the 14 genes had an AUROC of 0.8330 (figure 10). Removing the six least significant genes form the model, leaving eight genes in the panel (Model 7; MLH1, SEPT9v2, BNC1, ALX4, CDKN2B, NEUROG1, WNT5A, and TFPI2) resulted in an AUROC of 0.8211 (figure 10 and 11). Model 7 was determined as the model with best performance to differentiate between stage I or II and stage III or IV pancreatic cancer (probability cut point of 0.66; sensitivity 72.73% and specificity 80%). There were no statistical significant interactions between variables in model 7. The model was well calibrated (p=0.2750) and had an estimated optimism in AUROC of 0.06.

### Stage I, IIa vs IIb

It was not possible to differentiate pancreatic cancer patients with lymph node metastasis (stage IIb) from patients without lymph node metastasis (stage I or IIa) based on the hypermethylation profile.

### Stage I, II vs III

The hypermethylation profile was not able to differentiate unresectable primary tumor (T4) from potentially resectable primary tumors (T1-T3).

### Discussion

We examined cell-free DNA promoter hypermethylation of 28 genes in plasma of patients with pancreatic cancer and show that alteration in cell-free DNA hypermethylation is detectable in all cancer stages,which is consistent with previous studies. In our study patients with stage IV disease stands out by having significantly higher number of hypermethylated genes in cell-free DNA than stage I, II and III disease. This may be caused by distant metastasis resulting in larger amount of cell-free DNA and an accumulation of epigenetic changes during cancer development. The association between more advanced pancreatic cancer stage and higher number of hypermethylated genes in cell-free DNA, has not previously been described. Two small studies on cell-free DNA hypermethylation in pancreatic cancer were not able to show this association. Sato et al. demonstrated the presence of aberrant methylations in pancreatic tissue from early precursor lesions (pancreatic intraductal neoplasia (PanIN-1)), and found an increase in methylation prevalence from PanIN-1 to PanIN-3, suggesting DNA hypermethylations progressively increase during neoplastic progression. Our study is not able to demonstrate that detectable epigenetic alteration in cell-free DNA also accumulate from stage I to stage III disease, which probably is due to insufficient power.

We found that cell-free DNA hypermethylation of seven genes (ALX4, BNC1, HIC1, SEPT9v2, SST, TFPI2, and TAC1) was significantly associated with pancreatic cancer with distant metastases. This has not previously been described. Zhao et al. found HIC1 hypermethylation to be significantly higher in pancreatic cancer tissue stage III and IV compared to stage I and II. Cell-free DNA hypermethylation of TFPI2 has also been associated with stage four colorectal cancer. In contrast to pancreatic cancer, hypermethylation of ALX4 and SEPT9v2 has been found at same frequency in all stages of colorectal cancer tissue. In addition, SEPT9v2 hypermethylation was detectable in cell-free DNA of colorectal cancer patients in all stages, suggesting SEPT9v2 not to be associated with distant metastases in colorectal cancer.

A recent study on colorectal cancer described a panel of ten genes (including BNC1) which was more frequently hypermethylated in tissue from adenomas and early stage colorectal cancer compared to tissue from metastatic colorectal cancer. This suggests that hypermethylations not only accumulate, but also changes throughout cancer progression. In this study we developed two gene panels; a panel to distinguish stage I, II, III from IV and a panel to distinguish stage I, II from III and IV. The panels overlap with five genes out of eight, indicating that specific hypermethylations are of importance at different cancer stages. We previously analyzed the same 28 genes as a diagnostic marker for pancreatic cancer, and found an alternative gene panel to differentiate between patients with benign and malign pancreatic disease. Only BNC1 was recurring in the diagnostic and prognostic gene panels. This again supports the hypothesis that alteration in DNA hypermethylation are changing during the cause of pancreatic cancer.

We developed two prognostic markers, which are able to differentiate patients with pancreatic cancer according to staging. In particular, the gene panel to distinguish patients with distant metastasis (stage IV) from patients without distant metastasis (stage I, II and III), has a very high performance. To our knowledge, this is the first time prognostic prediction models based on alterations in cell-free DNA hypermethylation have been described regarding pancreatic cancer. The gene panels have the potential to be used as supplements to existing diagnostic tools in stage classification of patients with pancreatic cancer. The gene panels are blood based markers which are minimal invasive and thereby of great benefit to the patients. In addition, the analytical method is not a time consuming procedure, but can be performed in only two hours. Prognostic markers based on cell-free DNA hypermethylation are not depending on blood group status like CA-19-9,(9) which is a significant advantage. However, extern validation is needed before the prognostic markers can be applied in a clinical setting.

Some of the groups contained limited numbers of patients. This could result in lacking power, and hence, lack of difference in methylation profile between stage I, IIa vs IIb and stage I, II vs III. We discovered in the end of the analysis that the use of UNG (Invitrogen) had a tendency to lower the sensitivity compared to the use of COD UNG (ArcticZymes). All the samples in our study were analyzed using UNG (Invitrogen). Due to lack of sample material it was impossible to repeat the analysis with COD UNG (ArcticZymes). Still, plasma derived cell-free DNA hypermethylation of a broad gene panel was tested in a large group of patients all having pancreatic adenocarcinoma. Patients were included prospectively and consecutively. Blood samples were obtained before the diagnosis and any treatment. All patients had a systematic and comprehensive diagnostic work up to ensure the correct diagnosis and stage classification. Methylation specific PCR with an optimized method of bisulfite treatment was performed, which has an improved sensitivity compared to previous methods.

### Conclusion

Our study shows detectable alterations in DNA hypermethylation of plasma derived cell-free DNA even in early stage pancreatic cancer. The hypermethylations accumulate and change during the neoplastic development and with aggravating cancer stage. With high performance, panels of genes are able to differentiate pancreatic cancer patients according to cancer stage. Based on our study, alterations in cell-free DNA hypermethylation have the potential of being blood based prognostic markers for pancreatic cancer and a supplement to existing clinical tools in stage classification.

## Claims

1. A method of determining pancreatic cancer in a human subject, said method comprising in cell-free DNA from a blood sample from said human subject determining the methylation status of the gene loci BMP3, RASSF1A, BNC1, MESTv2, TFPI2, APC, SFRP1 and SFRP2.

2. A method for assessing whether a human subject has pancreatic cancer, said method comprising
a) providing a blood sample from said human subject,
b) isolating cell-free DNA from said blood sample,
c) determining in said sample the methylation status of the gene loci BMP3, RASSF1A, BNC1, MESTv2, TFPI2, APC, SFRP1 and SFRP2,
d) on the basis of said methylation status identifying a human subject that has pancreatic cancer.

3. The method according to any of the preceding claims, wherein said blood sample is a plasma sample.

4. The method according to any of the preceding claims, wherein the presence of methylated nucleic acid alleles are indicative of pancreatic cancer.

5. The method according to any of the preceding claims, wherein said methylation status is determined by Methylation-Specific PCR (MSP).

6. The method according to any of the preceding claims, wherein said methylation status is determined by a method comprising the steps of
i) providing a blood sample from said subject,
ii) isolating nucleic acid material, such as cell free DNA, from said blood sample,
iii) modifying said nucleic acid using an agent which modifies unmethylated cytosine,
iv) amplifying a portion of said gene loci using primers, which specifically recognize modified nucleic acid alleles and/or primers, which specifically recognize unmodified nucleic acid alleles, thereby obtaining an amplification product, and
v) detecting the amount of amplification product generated from primers specific for modified nucleic acid alleles and/or unmodified nucleic acid alleles.

7. The method according to any of the preceding claims, wherein said methylation status is determined by amplifying at least one portion of said gene loci using at least one primer pair selected from the nucleic acid sequences set forth in table 2 (SEQ ID NO: SEQ ID NO: 1-135).

8. The method according to any of the preceding claims, wherein said methylation status is determined by amplifying at least one portion of said gene loci, and wherein the amplified portion is detected using at least one oligonucleotide probe, wherein said primer and/or probe is selected from the primers and probes set forth in table 2.

9. Use of an oligonucleotide primer comprising a sequence, which is a subsequence of a gene locus selected from the group consisting of BMP3, RASSF1A, BNC1, MESTv2, TFPI2, APC, SFRP1 and SFRP2 or the complement thereof for diagnosing pancreatic cancer in a method of any of the preceding claims.

10. The use according to claim 9, wherein said oligonucleotide primer is selected from the nucleic acid sequences set forth in table 2 (SEQ ID NO: SEQ ID NO: 1-135).

## Patentansprüche

1. Verfahren zum Bestimmen von Bauchspeicheldrüsenkrebs bei einem menschlichen Individuum, wobei das Verfahren in zellfreier DNA aus einer Blutprobe von dem menschlichen Individuum Bestimmen des Methylierungsstatus der Genloci BMP3, RASSF1A, BNC1, MESTv2, TFPI2, APC, SFRP1 und SFRP2 umfasst.

2. Verfahren zum Beurteilen, ob ein menschliches Individuum Bauchspeicheldrüsenkrebs hat, wobei das Verfahren umfasst
a) Bereitstellen einer Blutprobe von dem menschlichen Individuum,
b) Isolieren von zellfreier DNA aus der Blutprobe,
c) Bestimmen des Methylierungsstatus der Genloci BMP3, RASSF1A, BNC1, MESTv2, TFPI2, APC, SFRP1 und SFRP2 in der Probe,
d) auf der Grundlage des Methylierungsstatus Identifizieren eines menschlichen Individuums, das Bauchspeicheldrüsenkrebs hat.

3. Verfahren nach einem der vorhergehenden Ansprüche, wobei die Blutprobe eine Plasmaprobe ist.

4. Verfahren nach einem der vorhergehenden Ansprüche, wobei das Vorhandensein von Allelen mit methylierter Nukleinsäure auf Bauchspeicheldrüsenkrebs hinweist.

5. Verfahren nach einem der vorhergehenden Ansprüche, wobei der Methylierungsstatus durch methylierungsspezifische PCR (MSP) bestimmt wird.

6. Verfahren nach einem der vorhergehenden Ansprüche, wobei der Methylierungsstatus durch ein Verfahren bestimmt wird, das die folgenden Schritte umfasst
i) Bereitstellen einer Blutprobe von dem Individuum,
ii) Isolieren von Nukleinsäurematerial, wie etwa zellfreier DNA, aus der Blutprobe,
iii) Modifizieren der Nukleinsäure unter Verwendung eines Mittels, das unmethyliertes Cytosin modifiziert,
iv) Amplifizieren eines Teils der Genloci unter Verwendung von Primern, die spezifisch Allele mit modifizierter Nukleinsäure erkennen, und/oder Primern, die spezifisch Allele mit unmodifizierter Nukleinsäure erkennen, dadurch Erhalten eines Amplifikationsprodukts, und
v) Nachweisen der Menge an Amplifikationsprodukt, das von Primern erzeugt wird, die für Allele mit modifizierter Nukleinsäure und/oder Allele mit unmodifizierter Nukleinsäure spezifisch sind.

7. Verfahren nach einem der vorhergehenden Ansprüche, wobei der Methylierungsstatus durch Amplifizieren mindestens eines Teils der Genloci unter Verwendung mindestens eines Primerpaars bestimmt wird, das aus den in Tabelle 2 (SEQ ID NO: SEQ ID NO: 1-135) aufgeführten Nukleinsäuresequenzen ausgewählt ist.

8. Verfahren nach einem der vorhergehenden Ansprüche, wobei der Methylierungsstatus durch Amplifizieren mindestens eines Teils der Genloci bestimmt wird und wobei der amplifizierte Teil unter Verwendung mindestens einer Oligonukleotid-Sonde nachgewiesen wird, wobei der Primer und/oder die Sonde aus den in Tabelle 2 aufgeführten Primern und Sonden ausgewählt ist.

9. Verwendung eines Oligonukleotid-Primers, der eine Sequenz umfasst, die eine Teilsequenz eines Genlocus, der aus der Gruppe ausgewählt ist, die aus BMP3, RASSF1A, BNC1, MESTv2, TFPI2, APC, SFRP1 und SFRP2 besteht, oder das Komplement davon ist, zum Diagnostizieren von Bauchspeicheldrüsenkrebs in einem Verfahren nach einem der vorhergehenden Ansprüche.

10. Verwendung nach Anspruch 9, wobei der Oligonukleotid-Primer aus den in Tabelle 2 (SEQ ID NO: SEQ ID NO: 1-135) aufgeführten Nukleinsäuresequenzen ausgewählt ist.

## Revendications

1. Procédé de détermination du cancer du pancréas chez un sujet humain, ledit procédé comprenant de l'ADN acellulaire provenant d'un échantillon de sang dudit sujet humain déterminant l'état de méthylation des loci des gènes BMP3, RASSF1A, BNC1, MESTv2, TFPI2, APC, SFRP1 et SFRP2.

2. Procédé pour évaluer si un sujet humain est atteint d'un cancer du pancréas, ledit procédé comprenant
a) la fourniture d'un échantillon de sang dudit sujet humain,
b) l'isolation de l'ADN acellulaire dudit échantillon de sang,
c) la détermination dans ledit échantillon de l'état de méthylation des loci des gènes BMP3, RASSF1A, BNC1, MESTv2, TFPI2, APC, SFRP1 et SFRP2,
d) sur la base dudit état de méthylation, l'identification d'un sujet humain qui a un cancer du pancréas.

3. Procédé selon l'une quelconque des revendications précédentes, dans lequel ledit échantillon de sang est un échantillon de plasma.

4. Procédé selon l'une quelconque des revendications précédentes, dans lequel la présence d'allèles d'acides nucléiques méthylés est indicative d'un cancer du pancréas.

5. Procédé selon l'une quelconque des revendications précédentes, dans lequel ledit état de méthylation est déterminé par PCR spécifique à la méthylation (MSP).

6. Procédé selon l'une quelconque des revendications précédentes, dans lequel ledit état de méthylation est déterminé par un procédé comprenant les étapes de
i) fourniture d'un échantillon de sang dudit sujet humain,
ii) isolation du matériel d'acide nucléique, tel que l'ADN acellulaire, dudit échantillon de sang,
iii) modification dudit acide nucléique à l'aide d'un agent modifiant la cytosine non méthylée,
iv) amplification d'une portion desdits loci de gènes à l'aide d'amorces, qui reconnaissent spécifiquement les allèles d'acides nucléiques modifiés et/ou d'amorces, qui reconnaissent spécifiquement les allèles d'acides nucléiques non modifiés, obtenant ainsi un produit d'amplification, et
v) détection de la quantité de produit d'amplification généré à partir d'amorces spécifiques d'allèles d'acides nucléiques modifiés et/ou d'allèles d'acides nucléiques non modifiés.

7. Procédé selon l'une quelconque des revendications précédentes, dans lequel ledit état de méthylation est déterminé en amplifiant au moins une portion desdits loci de gènes à l'aide d'au moins une paire d'amorces choisie parmi les séquences d'acides nucléiques présentées dans le tableau 2 (SEQ ID NO: SEQ ID NO: 1-135).

8. Procédé selon l'une quelconque des revendications précédentes, dans lequel ledit état de méthylation est déterminé en amplifiant au moins une partie desdits loci de gènes, et dans lequel la partie amplifiée est détectée à l'aide d'au moins une sonde oligonucléotidique, dans lequel ladite amorce et/ou sonde est choisie parmi les amorces et les sondes présentées dans le tableau 2.

9. Utilisation d'une amorce oligonucléotidique comprenant une séquence, qui est une sous-séquence d'un locus de gène choisi dans le groupe constitué de BMP3, RASSF1A, BNC1, MESTv2, TFPI2, APC, SFRP1 et SFRP2 ou leur complément pour diagnostiquer le cancer du pancréas dans un procédé selon l'une quelconque des revendications précédentes.

10. Utilisation selon la revendication 9, dans laquelle ladite amorce oligonucléotidique est choisie parmi les séquences d'acides nucléiques indiquées dans le tableau 2 (SEQ ID NO: SEQ ID NO: 1-135).
